(19) Europäisches Patentamt
European Patent Office
Office européen des brevets

(11) Publication number: **0 410 411 A2**

# (12) EUROPEAN PATENT APPLICATION

(21) Application number: 90114250.5

(22) Date of filing: 25.07.90

(51) Int. Cl.5: C07K 5/04, A61K 37/64, C07C 229/08, C07C 229/36

(30) Priority: 26.07.89 US 385624

(43) Date of publication of application: 30.01.91 Bulletin 91/05

(84) Designated Contracting States: AT BE CH DE DK ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Bey, Philippe
7875 Ivygate Lane
Cincinnati Ohio 45242(US)
Inventor: Peet, Norton P.
8028 Chestershire Drive
Cincinnati Ohio 45241(US)
Inventor: Angelastro, Michael R.
3018 Stratford Court
Loveland, Ohio 45140(US)
Inventor: Mehdi, Shujaath
6430 Welton St.
Cincinnati, Ohio 45213(US)

(74) Representative: Vossius & Partnerner
Siebertstrasse 4 P.O. Box 86 07 67
D-8000 München 86(DE)

## (54) Novel peptidase inhibitors.

(57) This invention relates to activated electrophilic ketone analogs of certain peptidase substrates which are useful in inhibiting serine-, carboxylic acid- and metallo- proteolytic enzymes, the inhibition of which will have useful physiological consequences in a variety of disease states.

EP 0 410 411 A2

## NOVEL PEPTIDASE INHIBITORS

This invention relates to protease enzyme inhibitors useful for a variety of physiological end-use applications.

In its broad aspects, this invention relates to analogs of peptidase substrates in which the carboxy terminal carboxy group has been replaced by a pentafluoroethylcarbonyl ($-C(O)C_2F_5$)group. These peptidase substrate analogs provide specific enzyme inhibitors for a variety of proteases, the inhibition of which exert valuable pharmacological activities and therefore have useful physiological consequences in a variety of disease states.

In its more specific aspects, this invention relates to pentafluoroethylcarbonyl analogs of certain peptidase substrates which are useful in inhibiting serine-, carboxylic acid- and metallo-proteinases, the inhibition of which will have useful physiological consequences in a variety of disease states.

Still more specifically, this invention relates to pentafluoroethylcarbonyl analogs of peptidase substrates which fall within the following generic groupings characterized according to their active site dependencies. Such generic groupings are:

I. Serine Proteinases: These include such enzymes such as Elastase (human leukocyte), Cathepsin G, Thrombin, Plasmin, C-1 Esterase, C-3 Convertase, Urokinase, Plasminogen Activator, Acrosin, β-Lactamase, D-Alanine-D-Alanine Carboxypeptidase, Chymotrypsin, Trypsin and Kallikreins.

II. Carboxylic Acid Proteinases: These include such specific enzymes as Renin, Pepsin and Cathepsin D.

III. Metallo Proteinases: These include Angiotensin Converting Enzyme, Enkephalinase, Pseudomonas Elastase and Leucine Aminopeptidase.

The contemplated peptidase inhibitors of the foregoing enzymes are selected from the generic formula

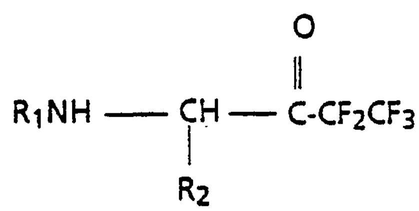

1

the hydrates, isosteres or the pharmaceutically acceptable salts thereof wherein:

$R_1$ is hydrogen, an amino protecting group selected from Group K, an α-amino acid or a peptide comprised of a number of α-amino acids, each of said α-amino acids or peptide optionally bearing an amino protecting group preferably selected from Group K,

$R_2$ is the side chain of the α-amino acid building block responsible for directing the inhibitor to the active site of the enzyme.

Isosteres of the compounds of formula I include those wherein (a) one or more of the α-amino residues of the $R_1$ substituent is in its unnatural configuration (when there is a natural configuration) or (b) when the normal peptidic amide linkage is modified, such as for example, to form $-CH_2NH-$ (reduced), $-COCH_2-$ (keto), $-CH(OH)CH_2-$ (hydroxy), $-CH(NH_2)CH_2-$ (amino), $-CH_2CH_2-$ (hydrocarbon). Preferably a compound of the invention should not be in an isosteric form, particularly it is preferred that there be no modified peptidic amide group in the $R_1$ group, but if there is, it is preferable to keep the isosteric modifications to a minimum.

Unless otherwise stated, the α-amino acids of these peptidase substrate analogs are preferably in their L-configuration. In referring to specific amino acids using the well known three letter codes, those amino acids in the L-configuration will be denoted by use of a capital letter for the first letter of the code and those amino acids of the D-configuration will be denoted by use of a lower case letter for the first letter of the code.

Those compounds of this invention having aspartic or glutamic acid moieties may be in free form or a salt form, e.g., acid addition or anionic salt. Such a compound may be converted into its salt or base form in an art-known manner, one from another. Preferred salts are trifluoroacetate, hydrochloride, sodium, potassium, or ammonium salts, although the scope of salts embraced herein is not limited thereto, the scope being extended to include all of the salts known to be used in the art of peptide chemistry.

Before further defining and/or illustrating the scope of the peptidase inhibitors embraced by formula I, it may be convenient to state some of the more basic concepts related to peptides. Each α-amino acid has a

characteristic "R-group", the R-group being the side chain, or residue, attached to the α-carbon atom of the α-amino acid. For example, the R-group side chain for glycine is hydrogen, for alanine it is methyl, for valine it is isopropyl. For the specific R-groups - or side chains - of the α-amino acids reference to A.L. Lehninger's text on Biochemistry (see particularly Chapter 4) is helpful.

As a further convenience for defining the scope of the compounds embraced by the generic concept of formula I, as well as the sub-generic concepts relating to each of the individual enzymes involved in this invention, various α-amino acids have been classified into a variety of groups which impart similar functional characteristics for each of the specific enzymes to be inhibited by the peptidase substrates of formula I. These groups are set forth in Table II and the recognized abbreviations for the α-amino acids are set forth in Table I.

| TABLE I | |
|---|---|
| AMINO ACID | SYMBOL |
| Alanine | Ala |
| Arginine | Arg |
| Aspargine | Asn |
| Aspartic acid | Asp |
| Asn + Asp | Asx |
| Cysteine | Cys |
| Glutamine | Gln |
| Glutamic acid | Glu |
| Gln + Glu | Glx |
| Glycine | Gly |
| Histidine | His |
| Isoleucine | Ile |
| Leucine | Leu |
| Lysine | Lys |
| Methionine | Met |
| Phenylalanine | Phe |
| p-Guanidinophenylalanine | Phe(Gua) |
| Proline | Pro |
| Serine | Ser |
| Threonine | Thr |
| Tryptophan | Trp |
| Tyrosine | Tyr |
| Valine | Val |
| Norvaline | Nva |
| Norleucine | Nle |
| 1-Naphthylalanine | Nal(1) |
| 2-Indolinecarboxylic acid | Ind |

| TABLE I | |
|---|---|
| **AMINO ACID** | **SYMBOL** |
| Sarcosine | Sar |
| Cyclohexylalanine | Cha |
| beta-Alanine | bAla |
| beta-Valine | bVal |
| O-4'-Methyltyrosine | Tyr(Me) |
| 3-Pyrazolylalanine | Ala(3pyr) |
| 4-Pyrimidinylalanine | Ala(4pyr) |
| $N^6$-(2-Carboxybenzoyl)lysine | Lys(2CBz) |
| Terephtholyl | tPht |
| $N^6$-Acetyllysine | Lys(Ac) |

TABLE II

Group A: Lys and Arg
B: Glu, Asp
C: Ser, Thr, Gln, Asn, Cys, His, Ala(3-pyr), Ala, (4-pyr) and N-methyl derivatives
D: Pro, Ind
E: Ala, bAla, Leu, Ile, Val, Nva, bVal, Met, and N-methyl derivatives
F: Phe, Tyr, Tyr(Me), Ala(3pyr), Ala(4-pyr), Trp, Nal(I), and N-methyl derivatives
G: Gly, Sar

J:

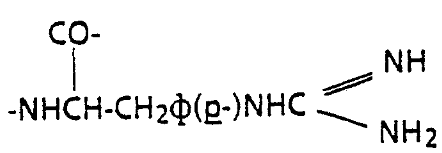 (J-1), 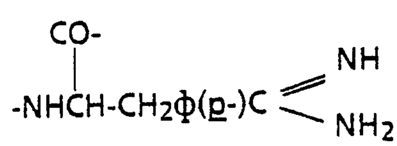 (J-2),

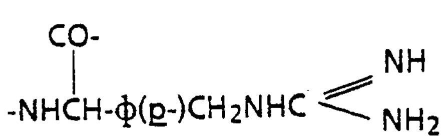 (J-3), and 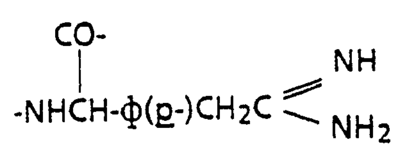 (J-4)

with Φ -representing phenyl

K: Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulphonyl ($AdSO_2$), 1-Adamantaneacetyl (AdAc), 2-Carboxybenzoyl (2Cbz), Phenylacetyl (PhAc), t -Butylacetyl (Tba), bis[(1-naphthyl)-methyl]acetyl (BNMA),

or -A-$R_z$ wherein

A is 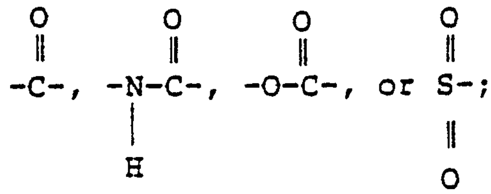

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl "Sac") containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

In light of the foregoing, the defined compounds of formula I may also be stated as being

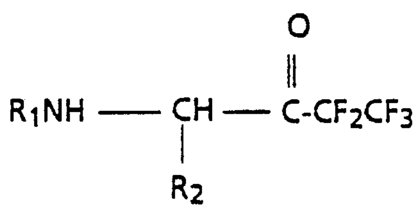

1

the hydrates, isosteres or the pharmaceutically acceptable salts thereof wherein:

$R_1$ is hydrogen, an amino protecting group selected from Group K, an $\alpha$-amino acid or a peptide comprised of a number of $\alpha$-amino acids, each of said $\alpha$-amino acids or peptide optionally bearing an amino protecting group preferably selected from Group K,

$R_2$ is the side chain of the $\alpha$-amino acid responsible for directing the inhibitor to the active site of the enzyme

wherein the said $\alpha$-amino acid and peptide moieties are selected from Groups A, B, C, D, E, F, G and J, and K is a terminal amino protecting group, members of these groups being

Group A: Lys and Arg

B: Glu, Asp

C: Ser, Thr, Gln, Asn, Cys, His, and N-methyl derivatives

D: Pro, Ind

E: Ala, bAla, Leu, Ile, Val, Nva, bVal, Met, Nle and N-methyl derivatives

F: Phe, Tyr, Trp, Nal(I), and N-methyl derivatives G: Gly, Sar

J:

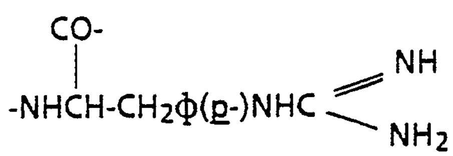 (J-1), 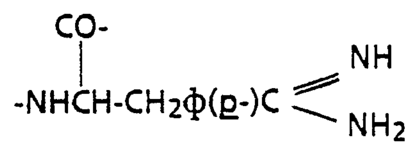 (J-2),

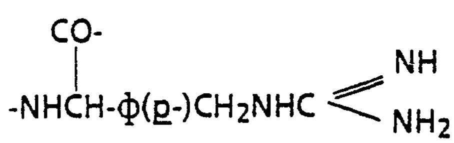 (J-3), and 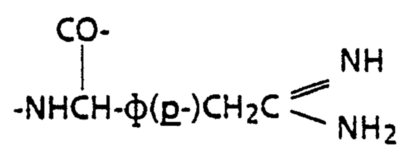 (J-4)

with Φ representing phenyl

K. Acetyl (Ac), Succinyl (Suc), Benzoyl (Bz), t -Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulphonyl ($AdSO_2$), 1-Adamantaneacetyl (AdAc), 2-Carboxybenzoyl (2Cbz), Phenylacetyl (PhAc), t -Butylacetyl (Tba), bis[(1-naphthyl)-methyl]acetyl (BNMA),

or $-A-R_z$ wherein

A is 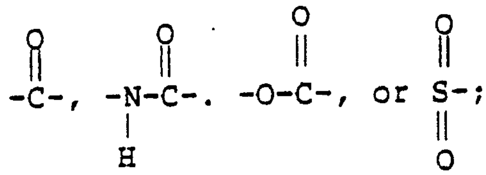

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl) containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

The compounds of formula I can also be depicted as a peptide derivative, albeit modified on its carboxy terminal end. In this depiction the $R_2$ moiety is in the $P_1$ position of the peptide, the α-amino acids of the $R_1$ moiety would be in the $P_2 \rightarrow P_n$ positions, n being the numeric sequence dependent upon the number of α-amino acid building blocks in that particular compound, e.g., if $R_1$ contained four α-amino acids it would be comprised of $P_2$-$P_3$-$P_4$-$P_5$ positions with the option of a terminal amino protecting group from Group K in the $P_5$ moiety.

To further illustrate the shorthand nomenclature used throughout this application assume that $R_1$ is comprised of $P_2$, $P_3$, $P_4$ having a terminal amino protecting group so that $R_1$ is $-Pro-Ala-Ala-Suc-OCH_3$, $R_2$ is isopropyl, then that specific compound would be written as $H_3CO$-Suc-Ala-Ala-Pro-Val.

It is also to be noted that in some instances it is more convenient to designate the terminal amino protecting group as a separate $P_n$ position of the peptide. The terminal amino protecting group would be designated as being in the $P_5$ position and thus $R_1$ would be $P_2$-$P_3$-$P_4$-$P_5$ with $P_5$ being a protecting group of Group K. If $P_4$ optionally is deleted, then quite obviously, when $P_4$ is deleted the protecting group of $P_5$ would be attached to the $P_3$ moiety. In those instances wherein Group K represents an $-A-R_z$ moiety, it is preferred that A represent -C(=O)- and that $R_z$ represent acylsulfonamido, particularly those wherein the acylsulfonamido contains an aryl moiety (preferably phenyl) substituted by a halogen, the preferred $-A-R_z$ moieties being 4-[(4-chlorophenyl)sulfonylaminocarbonyl]phenylcarbonyl, 4-[(4-bromophenyl)-sulfonylaminocarbonyl]phenylcarbonyl and 4-[phenylsulfonylaminocarbonyl] phenylcarbonyl (said moieties being abbreviated as ClΦSacBz, BrΦSacBz and ΦSacBz, respectively).

Utilizing the foregoing illustrations those compounds of formula I which are useful as inhibitors for human leukocyte elastase are represented by the formula

**$R_1NHCH(R_2)COCF_2CF_3$** Ia

and the hydrates, isosteres or the pharmaceutically acceptable salts thereof, wherein

$R_1$ is $P_2$-$P_3$-$P_4$-$P_5$ with

$P_2$ being an α-amino acid selected from Groups D, E and F, with proline being preferred,

$P_3$ is an α-amino acid of Groups D, E, or lysine with isoleucine, valine or alanine being preferred,

$P_4$ is an α-amino acid of Groups E or zero with alanine being preferred (when $P_n$ is zero then that particular moiety does not appear in the structure, i.e., it is deleted), and

$P_5$ is a terminal moiety of Group K with methoxysuccinyl and Cbz and ClΦSacBz, BrΦSacBz and ΦSacBz being preferred, and

$R_2$ is the side chain of an amino acid of Groups E and G, with the side chain of nor-valine and valine being preferred.

Human leukocyte elastase is released by polymorphonuclear leukocytes at sites of inflammation and thus is a contributing cause for a number of disease states. Thus the peptidase substrates of formula (Ia) have an anti-inflammatory effect useful in the treatment of gout, rheumatoid arthritis and other inflammatory diseases, and in the treatment of emphysema. In their end-use application the enzyme inhibitory properties

of the compounds of (Ia) are readily ascertained by standard biochemical techniques well known in the art. Potential dose range for their end-use application will of course depend upon the nature and severity of the disease state as determined by the attending diagnostician with the range of 0.01 to 10 mg/kg body weight per day being useful for the aforementioned disease states with 0.1 mg to 10 mg/kg per day being preferred. The preferred compounds for this enzyme are:

MeOSuc-Ala-Ala-Pro-Val-$C_2F_5$,
$AdSO_2$-Lys(2Cbz)-Pro-Val-$C_2F_5$,
Cbz-Val-Pro-Val-$C_2F_5$,
ClΦ$S_{AC}$Bz-Val-Pro-Val-$C_2F_5$,
BrΦSacBz-Val-Pro-Val-$C_2F_5$,
Φ-SacBz-Val-Pro-Val-$C_2F_5$,
tPht-Val-Pro-Val-$C_2F_5$, and
Boc-Val-Pro-Val-$C_2F_5$.

Those compounds of formula I which are useful as inhibitors of Cathepsin G are represented by the structural formula

**$R_1NHCH(R_2)COCF_2CF_3$** Ib

and the hydrates, isosteres or the pharmaceutically acceptable salts thereof, wherein
$R_1$ is -$P_2$-$P_3$-$P_4$-$P_5$ with
$P_2$ being selected from Groups D, E, or G, with proline being preferred,
$P_3$ is selected from Groups E or G with alanine and valine being preferred,
$P_4$ is selected from Groups E, G or is deleted with alanine being preferred, the terminal α-amino acid optionally bearing a protecting group selected from Group K with succinyl ClΦSacBz or other SAC containing groups or methoxy succinyl being preferred, and
$R_2$ is selected from side chains of the amino acids of Groups E and F but preferably is benzyl.

The end-use application of the compounds (Ib) inhibiting Cathepsin G is the same as for human leukocyte inhibitors, including arthritis, gout and emphysema, but also embracing the treatment of glomerulonephritis and lung infestations caused by infections in the lungs. For their end-use application, the potency and other biochemical parameters of the enzyme inhibiting characteristics of the compounds of (Ib) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 to 10 mg/kg per day being preferred. Preferred compounds for formula (Ib) are:

MeOSuc-Ala-Ala-Pro-Phe-$C_2F_5$,
Suc-Ala-Ala-Pro-Phe-$C_2F_5$, and
ClΦSacBz-Val-Pro-Phe-$C_2F_5$.

Those compounds of formula I which are useful as inhibitors of thrombin are represented by the formula

**$R_1NHCH(R_2)COCF_2CF_3$** Ic

and the hydrates, isosteres or the pharmaceutically acceptable salts thereof, wherein
$R_1$ is -$P_2$-$P_3$, (b) -$P_2$ or (c) -$P_2$-$P_3$-$P_4$ wherein:

(a) $P_2$ is selected from Groups D, E or F, preferably proline, $P_3$ is selected from Group F, each $P_3$ is selected from Group F, each $P_3$ being in the D-configuration, preferably phe,

(b) $P_2$ is selected from Group K but preferably is dansyl, tosyl or benzoyl,

(c) $P_2$ is selected from Group E but preferably is alanine, $P_3$ is selected from Groups C, G and E but preferably is serine, $P_4$ is selected from Groups F, G and E or is zero but preferably is Phe, and $R_2$ is preferably the arginine side chain but may also be selected from side chains of the amino acids of Groups A and J, preferably (J-1).

The compounds embraced by formula (Ic) inhibit thrombin and therefore, as in the use of heparin, the compounds may be used as the initial anticoagulant agent in thrombophlebitis and coronary thrombosis. For their end-use application, the potency and other biochemical parameters of the enzyme inhibiting characteristics of the compounds of (Ic) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. Preferred compounds are as expressed for Cathepsin G and also include:

phe-Pro-NHCH(J-1)-$C_2F_5$,

phe-Pro-Arg-$C_2F_5$,
Dns-Arg-$C_2F_5$,
H-Phe-Ser-Ala-$C_2F_5$,
H-(D)-Phe-Pro-Lys-$C_2F_5$, and
Bz-NHCH(J-1)-$C_2F_5$.

The compounds of formula I which are useful as inhibitors of chymotrypsin are represented by the structural formula

**$R_1NHCH(R_2)COCF_2CF_3$** Id

and the hydrates, isosteres or the pharmaceutically acceptable salts thereof, wherein
$R_1$ is -$P_2$-$P_3$-$P_4$-$P_5$ with
$P_2$ being selected from Groups D, E, with Leu being preferred, G or K with benzoyl being preferred,
$P_3$ is selected from Groups E or G or K, with acetyl being preferred, or is deleted, with alanine being preferred,
$P_4$ is selected from Groups E or G or K or is deleted, with alanine being preferred, and
$P_5$ is selected from Group K with succinyl being preferred or is deleted, and
$R_2$ is selected from the side chains of the amino acids of Groups E and F but preferably is the Phe side chain or the Tyr side chain.

The end-use application of the compounds (Id) inhibiting chymotrypsin is in the treatment of pancreatitis. For their end-use application, the potency and other biochemical parameters of the enzyme inhibiting characteristics of the compounds of (Id) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. Preferred compounds are as expressed for Cathepsin G and also include:
Bz-Phe-$C_2F_5$,
Bz-Tyr-$C_2F_5$,
Ac-Leu-Phe-$C_2F_5$, and
Cbz-Phe-$C_2F_5$.

The compounds of formula I which are useful as inhibitors of trypsin are represented by the structural formula **$R_1NHCH(R_2)COCF_2CF_3$** Ie

and the hydrates, isosteres or the pharmaceutically acceptable salts thereof, wherein
$R_2$ is as defined in (Ic), and,
$R_1$ is selected from (a) -$P_2$-$P_3$, (b) -$P_2$ or (c) -$P_2$-$P_3$-$P_4$ with

(a) $P_2$ is selected from Groups "E or F but is preferably proline or alanine, $P_3$ is selected from Group F, (each being in the D configuration) but preferably is phe,

(b) $P_2$ is selected from Group K but preferably is dansyl, tosyl or benzoyl, and,

(c) $P_2$ is selected from Group D or E but preferably is proline or alanine, $P_3$ is selected from Groups G and E but preferably is serine, $P_4$ is selected from Groups G and E or is zero but preferably is Phe.

The end-use application of the compounds (Ie) inhibiting trypsin is in the treatment of pancreatitis. For their end-use application, the potency and other biochemical parameters of the enzyme inhibiting characteristics of the compounds of (Ie) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. The preferred compounds useful for inhibiting trypsin are the same as for the inhibitors of thrombin.

The compounds of formula I which are useful as inhibitors of plasmin are represented by the structural formula

**$R_1NHCH(R_2)COCF_2CF_3$** If

and the hydrates, isosteres or the pharmaceutically acceptable salts thereof, wherein
$R_1$ is -$P_2$-$P_3$-$P_4$ with
$P_2$ being selected from Group E or F but preferably is Ala or Phe,
$P_3$ is selected from Groups B, F or K but preferably is Glu or acetyl, and
$P_4$ is selected from Group K or is deleted but preferably is dansyl, and
$R_2$ is selected from a side chain of an amino acid of Groups A and J but preferably is the side chain of lysine or J-I.

The compounds embraced by formula (If) inhibit plasmin and are therefore antiproliferative agents

useful in treating excessive cell growth, particularly in the treatment of benign prostatic hypertrophy and prostatic carcinoma, and in the treatment of psoriasis. For their end-use application, the potency and other biochemical parameters of the enzyme inhibiting characteristics of the compounds of (If) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 to 10 mg/kg per day being preferred. The preferred compounds are:

Dns-Glu-Phe-Lys-$C_2F_5$,

Ac-Ala-NHCH(J-1)-$C_2F_5$, and

Ac-Ala-Lys-$C_2F_5$.

The compounds of formula I which are useful as inhibitors of $C_1$-esterase are represented by the structural formula **$R_1$NHCH($R_2$)COC$F_2$C$F_3$** Ig

and the hydrates, isosteres or the pharmaceutically acceptable salts thereof wherein

$R_1$ is -$P_2$-$P_3$ with $P_2$ being selected from Groups E, G, D, C, F, A or B with Ala being preferred, and

$P_3$ is selected from Group K with Cbz or acetyl being preferred, and

$R_2$ is selected from the side chain of an amino acid of Groups A and J, but preferably the side chain of Arg or (J-1).

The compounds embraced by formula (Ig) inhibit $C_1$-esterase and are therefore useful in treating systemic lupus, arthritis, autoimmune hemolytic anemia and glomerulonephritis. For their end-use application, the potency and other biochemical parameters of the enzyme inhibiting characteristics of the compounds of (Ig) is readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. The preferred compounds are:

Cbz-Ala-Arg-$C_2F_5$ and

Ac-Ala-NHCH(J-1)CO-$C_2F_5$.

The compounds of formula I which are useful as inhibitors of $C_3$-convertase are represented by the formula

**$R_1$NHCH($R_2$)COC$F_2$C$F_3$** Ih

and the hydrates, isosteres or the pharmaceutically acceptable salts thereof, wherein $R_1$ is -$P_2$-$P_3$-$P_4$ with

$P_2$ being selected from Groups E or F, with Ala being preferred,

$P_3$ is selected from Groups E or F with Leu being preferred, and

$P_4$ is selected from Group K with Bz being preferred, and

$R_2$ is selected from the side chain of an amino acid of Groups A or J, with Arg being preferred.

The compounds embraced by formula (Ih) inhibit $C_3$-convertase and are therefore useful in treating systemic lupus, arthritis, autoimmune hemolytic anemia and glomerulonephritis. For their end-use application, the potency and other biochemical parameters of the enzyme inhibiting characteristics of the compounds of (Ih) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. The preferred compound is:

Bz-Leu-Ala-Arg-$C_2F_5$.

The compounds of formula I which are useful as inhibitors of Urokinase are represented by the formula

**$R_1$NHCH($R_2$)COC$F_2$C$F_3$** Ii

and the hydrates, isosteres or the pharmaceutically acceptable salts thereof, wherein

$R_1$ is -$P_2$-$P_3$ with

$P_2$ being selected from Groups E and G with Ala and Gly being preferred and

$P_3$ is selected from Group B with Glu being preferred, and

$R_2$ is selected from the side chain of an amino acid of Groups A and J with the side chain of Arg being preferred.

The compounds embraced by formula (Ii) inhibit Urokinase and therefore are useful in treating excessive cell growth disease states. As such compounds are useful in the treatment of benign prostatic hypertrophy and prostatic carcinoma, the treatment of psoriasis, and in their use as abortifacients. For their end-use application, the potency and other biochemical parameters of the enzyme inhibiting characteristics of the compounds of (Ii) are readily ascertained by standard biochemical techniques well known in the art.

Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred. The preferred compounds are:

K-Glu-Gly-Arg-$C_2F_5$ and

K-Glu-Gly-Phe(Gua)-$C_2F_5$,

with K being a protecting group.

The compounds of formula I which are useful as inhibitors of plasminogen activator are represented by the structural formula

**$R_1NHCH(R_2)COCF_2CF_3$** Ij

and the hydrates, isosteres or the pharmaceutically acceptable salts thereof, wherein

$R_1$ is -$P_2$-$P_3$-$P_4$ wherein

$P_2$ is Gly,

$P_3$ is selected from Group B with Glu being preferred, and

$P_4$ preferably is dansyl but also selected from Group K and

$R_2$ is selected from a side chain of an amino acid of Groups A and J with the side chain of Arg being preferred.

Preferred compounds are:

Dns-Glu-Gly-Arg-$C_2F_5$ and

Dns-Glu-Gly-Phe(Gua)-$C_2F_5$.

The compounds of formula I which are useful as inhibitors of acrosin are represented by the structural formula

**$R_1NHCH(R_2)COCF_2CF_3$** Ik

and the hydrates, isosteres or the pharmaceutically acceptable salts thereof, wherein

$R_1$ is -$P_2$-$P_3$-$P_4$ with

$P_2$ being selected from Group E or K with Leu or benzoyl being preferred,

$P_3$ is selected from Group E with Leu being preferred or is deleted, and

$P_4$ is selected from Group K with Boc being preferred or is deleted, and

$R_2$ is selected from the side chains of the amino acids of Groups A and J with the side chains of Arg and NHCH(J-1)CO being preferred.

The preferred compounds are:

Boc-Leu-Leu-Arg-$C_2F_5$, Boc-Leu-Leu-Phe(Gua)-$C_2F_5$, and

Bz-NRCH(J-1)-$C_2F_5$.

The compounds of formula (Ik) are acrosin inhibitors and therefore are useful as anti-fertility agents in that they possess the characteristics of preventing sperm from penetrating an otherwise fertilizable egg. For their end-use application, the potency and other biochemical parameters of the enzyme inhibiting characteristics of the compounds of (Ik) are readily ascertained by standard biochemical techniques well known in the art. Actual dose ranges for their specific end-use application will, of course, depend upon the state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of $\beta$-lactamase are represented by the structural formula

**$R_1NHCH(R_2)COCF_2CF_3$** Il and the hydrates, isosteres or the pharmaceutically acceptable salts thereof wherein

$R_1$ is $P_2$,

$P_2$ being selected from Group K with $COCH_2\Phi$ and Bz being preferred, and

$R_2$ is selected from a side chain of an amino acid of Groups E, G and C with hydrogen being preferred.

The preferred compounds are:

$\Phi CH_2CONHCH_2CO$-$C_2F_5$, and

$\Phi CH_2CONHCH_2CHOH$-$C_2F_5$.

The compounds embraced by formula (I1) inhibit $\beta$-lactamase and therefore are useful in the potentiation of antibacterial agents, particularly the $\beta$-lactam antibacterials. For their end-use application, the potency and other biochemical parameters of the enzyme inhibiting characteristics of the compounds of (I1) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that

the general end-use application dose range will be about 0.01 to 10 mg/kg per day for an effective therapeutic effect with 0.1 mg to 10 mg/kg per day being preferred.

The compounds of formula I which are useful as inhibitors of D-Ala-D-Ala carboxypeptidase are represented by the structural formula

**$R_1NHCH(R_2)COCF_2CF_3$** Im

and the hydrates, isosteres or the pharmaceutically acceptable salts thereof, wherein

$R_1$ is $P_2$-$P_3$ with

$P_2$ being Lys(Ac) or is selected from Groups E and C with Lys(Ac) being preferred, and

$P_3$ is selected from Group K with Ac being preferred, and

$R_2$ is methyl such that $P_1$ is ala.

The preferred compound is:

Ac-Lys(Ac)-ala-$C_2F_5$.

The compounds of formula I which are useful as inhibitors of peptidyl-prolyl cis -trans isomerase (PPI) are represented by the structural formula

**$R_1NHCH(R_2)COCF_2CF_3$** In

and the hydrates, isosteres or the pharmaceutically acceptable salts thereof wherein

$R_1$ is -$P_2$-$P_3$ wherein

$P_2$ is selected from Group E with Ala being preferred, and

$R_2$ is selected from a side chain of an amino acid from Group E with the side chain of Ala being preferred.

$P_3$ is selected from Group K with MeOSuc being preferred.

The compounds of formula In are inhibitors of peptidyl-prolyl cis-trans isomerase and would therefore be expected to possess immunosuppressant activity. Immunosuppressants, like cyclosporin, can be used to, for example, lessen the rejection of transplanted tissue or organ by the immune system of the host.

The compounds of formula I which are useful as inhibitors of renin are represented by the structural formula

**$R_1NHCH(R_2)COCF_2CF_3$** Io

and the hydrates, isosteres or the pharmaceutically acceptable salts thereof wherein

$R_1$ is -$P_2$-$P_3$-$P_4$-$P_5$-$P_6$ wherein

$P_2$ is selected from Groups E, C or F with His, Nva, Ala(3pyr) or Ala(4pyr), and Nle being preferred,

$P_3$ is selected from Groups E or F or is deleted with Phe or Tyr(Me) being preferred,

$P_4$ is selected from Groups E, D, F or is deleted with Pro, bAla or bVal being preferred,

$P_5$ is selected from Groups E, C, F or is deleted with His being preferred, and

$P_6$ is selected from Group K with Boc, Cbz or Tba being preferred, or being BNMA when $P_3$, $P_4$, $P_5$ are deleted, or when $P_4$ is bVal or bAla, $P_5$ and $P_6$ are deleted, and

$R_2$ is selected from a side chain of an amino acid of Groups E or F or Cha with the side chain of Leu or Cha being preferred.

The preferred compounds are:

Cbz-Nal(I) -His-Leu-$C_2F_5$,
Cbz-Phe-His-Leu-$C_2F_5$,
Boc-Phe-Nva-Leu-$C_2F_5$,
Cbz-Phe-Nva-Leu-$C_2F_5$,
Boc-His-Pro-Phe-His-Leu-$C_2F_5$,
Cbz-Phe-His-Cha-$C_2F_5$,
Cbz-His-Leu-$C_2F_5$,
Boc-Phe-His-Leu-$C_2F_5$,
Boc-Phe-Nva-Cha-$C_2F_5$,
Boc-Tyr(Me)-Nva-Cha-$C_2F_5$,
Boc-Phe-Ala(3pyr)-Cha-$C_2F_5$,
Tba-Tyr(Me)-Nva-Cha-$C_2F_5$,
Tba-Tyr(Me)-Ala(4pyr)-Cha-$C_2F_5$,
bAla-Tyr(Me)-Nva-Cha-$C_2F_5$,
bVal-Tyr(Me)-Nva-Cha-$C_2F_5$,
bVal-Tyr(Me)-His-Cha-$C_2F_5$, and
bAla-Tyr(Me)-His-Cha-$C_2F_5$.

The compounds of formula (Io) inhibit renin and therefore are used as antihypertensive agents useful in treating hypertension. For their end-use application, the potency and other biochemical parameters of the enzyme inhibiting characteristics of the compounds of (Io) are readily ascertained by standard biochemical techniques well known in the art. Actual dose ranges for their specific end-use application will, of course,

depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of pepsin are represented by the structural formula **$R_1NHCH(R_2)COCF_2CF_3$** Ip

and the hydrates, isosteres or the pharmaceutically acceptable salts thereof wherein

$R_1$ is $-P_2-P_3-P_4$ with

$P_2$ being selected from Groups E or F with Val being preferred,

$P_3$ is selected from Groups E or F with Val being preferred or is deleted and

$P_4$ is selected from Group K, preferably Iva, and

$R_2$ is selected from a side chain of an amino acid of Groups E and F with the side chain of Leu being preferred.

The preferred compounds are:

Iva-Val-Leu-$C_2F_5$ and

Iva-Val-Val-Leu-$C_2F_5$.

The compounds of formula (Ip) inhibit pepsin and therefore exert an antiulcer effect useful in the treatment and prevention of ulcers. For their end-use application, the potency and other biochemical parameters of the enzyme inhibiting characteristics of the compounds of (Ip) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of Cathepsin D are represented by the structural formula

**$R_1NHCH(R_2)COCF_2CF_3$** Iq

and the hydrates, isosteres or the pharmaceutically acceptable salts thereof, wherein

$R_1$ is $-P_2-P_3-P_4$ with

$P_2$ being selected from Groups E and F, with Val or Ala being preferred,

$P_3$ is selected from Groups E and F or is deleted with Val being preferred, and

$P_4$ is selected from Group K with Cbz being preferred, and

$R_2$ is selected from a side chain of an amino acid of Groups E and F, with the side chain of Phe being preferred.

The preferred compounds are:

Cbz-Val-Val-Phe-$C_2F_5$,

Iva-Val-Ala-Phe-$C_2F_5$, and

Iva-Val-Phe-$C_2F_5$.

As inhibitors of Cathepsin D the compounds of formula (Iq) are useful for the same end-use applications set forth for human leukocyte elastase inhibitors (Ia) and are also useful as antidemyelinating agents useful to prevent and arrest nerve tissue damage. For their end-use application, the potency and other biochemical parameters of the enzyme inhibiting characteristics of the compounds of (In) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of angiotensin converting enzyme (ACE) are represented by the structural formula

**$R_1NHCH(R_2)COCF_2CF_3$** Ir

and the hydrates, isoteres or the pharmaceutically acceptable salts thereof, wherein

$R_1$ is selected from Group K with Bz being preferred, and

$R_2$ is selected from a side chain of an amino acid of Groups E, F and G with the side chain of Phe being preferred.

The preferred compounds are:

Bz-Phe-$C_2F_5$ and

Cbz-Phe-$C_2F_5$.

The compounds of formula (Ir) inhibit ACE and are therefore useful as antihypertensives for treating

hypertension. For their end-use application, the potency and other biochemical parameters of the enzyme inhibiting characteristics of the compounds of (Ir) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of enkephalinase are represented by the structural formula

**$R_1NHCH(R_2)COCF_2CF_3$** Is

and the hydrates, isosteres or the pharmaceutically acceptable salts thereof, wherein

$R_1$ is $-P_2-P_3$, with

$P_2$ being Gly and

$P_3$ being selected from Group F or is deleted with Tyr being preferred, and

$R_2$ is hydrogen.

The preferred compound is:

Tyr-Gly-Gly-$C_2F_5$.

The compounds of formula (Is) inhibit enkephalinase and therefore are useful as analgesics. For their end-use application, the potency and other biochemical parameters of the enzyme inhibiting characteristics of the compounds of (Is) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of pseudomonas elastase are represented by the structural formula

**$R_1NHCIH(R_2)COCF_2CF_3$** It

and the hydrates, isosteres or the pharmaceutically acceptable salts thereof, wherein

$R_1$ is $-P_2-P_3$ with

$P_2$ being selected from Group E with Ala being preferred, and

$P_3$ is selected from Group K with MeOSuc being preferred, and

$R_2$ is selected from a side chain of an amino acid of Groups E and G with the side chain of Ala being preferred.

The preferred compound is

MeOSuc-Ala-Ala-$C_2F_5$.

The compounds of formula (It) inhibit pseudomonas elastase and therefore are useful as antibacterial agents particularly useful against infections caused by pseudomonas bacteria. For their end-use application, the potency and other biochemical parameters of the enzyme inhibiting characteristics of the compounds of (It) are readily ascertained by standard biochemical techniques well known in the art. Actual dose ranges for their specific end-use application will, of course, depend upon the nature and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of leucine aminopeptidase are represented by the structural formula

**$R_1NHCH(R_2)COCF_2CF_3$** Iu

and the hydrates, isosteres or the pharmaceutically acceptable salts thereof, wherein

$R_1$ is hydrogen, and

$R_2$ is selected from a side chain of an amino acid of Groups A, B, E, F and J with the side chain of Phe, Leu, Glu and Arg being preferred.

The preferred compounds are:

Leu-$C_2F_5$ and

Phe-$C_2F_5$.

The compounds of formula (Iu) are inhibitors of leucine amino peptidase and therefore are useful as immunostimulants useful in conjunctive therapy in the treatment with other known anticancer agents. For their end-use application, the potency and other biochemical parameters of the enzyme inhibiting characteristics of the compounds of (Iu) are readily ascertained by standard biochemical techniques well known in the art. Actual dose ranges for their specific end-use application will, of course, depend upon the nature

and severity of the disease state of the patient or animal to be treated as determined by the attending diagnostician. It is to be expected that the general end-use application dose range will be about 0.01 to 10 mg/kg per day for an effective therapeutic effect with 0.1 mg to 10 mg/kg per day being preferred.

The compounds of formula I which are useful as inhibitors of kallikreins, tissue or plasma, are represented by the structural formula

$R_1NHCH(R_2)COCF_2CF_3$ Iv

and the hydrates, isosteres or the pharmaceutically acceptable salts thereof, wherein

$R_1$ is $-P_2-P_3$ with

$P_2$ being selected from Groups E and F with Phe being preferred,

$P_3$ being selected from Groups C, E or F, which may be in either the D- or L-configuration, and

$R_2$ preferably is the side chain of Arg or (J-1).

The preferred compounds of this formula are:

pro-Phe-Arg-$C_2F_5$,

pro-Phe-NHCH(J01)CO-$C_2F_5$.

The compounds of formula (Iv) are inhibitors of the kallikreins, tissue or plasma, and therefore inhibit kinin formations. Kinins, generally known to induce pain and vascular permeability associated with inflammation and infection, e.g., bacterial and viral. The inhibition of the kinin formation renders these compounds useful in the alleviation of pain and inflammation. Furthermore, these compounds are useful as male contraceptives in that they will dramatically interfere with normal sperm function. In their end-use application dose range will be about 0.01 to 10 mg/kg per day for an effective therapeutic effect with 0.1 mg to 10 mg/kg per day being preferred.

The compounds of Formula I which are of particular use as inhibitors of retroviral protease required for replication, particularly the HIV-1 and HIV-2 viral proteases, the viruses putatively responsible for causing AIDS (acquired immune deficiency syndrome) are those compounds of Formula (Iw) $\mathbf{R_1NHCH(R_2)\text{-}COCF_2CF_3}$ Iw

wherein

$R_1$ is $-P_2-P_3-P_4$ with

$P_2$ being selected from the Groups C, E, F and G, preferably Asn, Gln and Ala,

$P_3$ being selected from the Groups C, E, F and G, preferably Asn, Gln and Ala,

$P_4$ being selected from Group C, or being bAla or bVal, preferably Ser or Thr, and optionally bearing an amino protecting group of Group K,

$R_2$ is the side chain of an $\alpha$-amino acid of Groups F, E, or Cha with the side chains of Met, Tyr, Phe and Cha being preferred.

The preferred compounds are:

Ser-Gln-Asn-Tyr-$C_2F_5$,
Ser-Gln-Asn-Phe-$C_2F_5$,
Ser-Leu-Asn-Tyr-$C_2F_5$,
Ser-Leu-Asn-Phe-$C_2F_5$,
Thr-Gln-Asn-Tyr-$C_2F_5$,
Thr-Gln-Asn-Phe-$C_2F_5$,
Thr-Gln-Asn-Met-$C_2F_5$,
Thr-Leu-Asn-Tyr-$C_2F_5$,
Thr-Leu-Asn-Phe-$C_2F_5$,
Iva-Ser-Asn-Tyr-$C_2F_5$,
Iva-Ser-Asn-Phe-$C_2F_5$,
Ser-Gln-Asn-Met-$C_2F_5$,
Ser-Leu-Asn-Met-$C_2F_5$,
Thr-Gln-Asn-Met-$C_2F_5$,
Thr-Leu-Asn-Met-$C_2F_5$, and
Iva-Ser-Asn-Met-$C_2F_5$.

In their end-use application in the treatment of retroviral infections, the compounds of Formula (Ix] will be administered at about 1-100 mg per kg of body weight per day, preferably intravenously.

From the above, it is obvious that in all of the foregoing instances of (Ia) through (Iw), the definitions of $R_b$, $R_a$, X, n and Q are as defined in the generic formula I with the specific preferred embodiments being further illustrated for each group of enzyme inhibitors. Of course, it is also understood that in those instances wherein the carbonyl moiety of $P_1$ is in its reduced form, then such compounds are not hydrates.

Having defined the scope of the compounds within the generic invention and within the individual subgeneric groups for each of the individual enzymes, the manner in which such may be prepared will be

described and illustrated.

In general, the compounds of formula I may be prepared using standard chemical reactions analogously known in the art. The procedure for preparing the formula I compounds is outlined in Scheme A wherein $R_1$ and $R_2$ are as previously defined, and

Pg is an amino protecting group such as a carbamate, preferably a benzyloxycarbonyl (Cbz) group.

## Reaction Scheme A

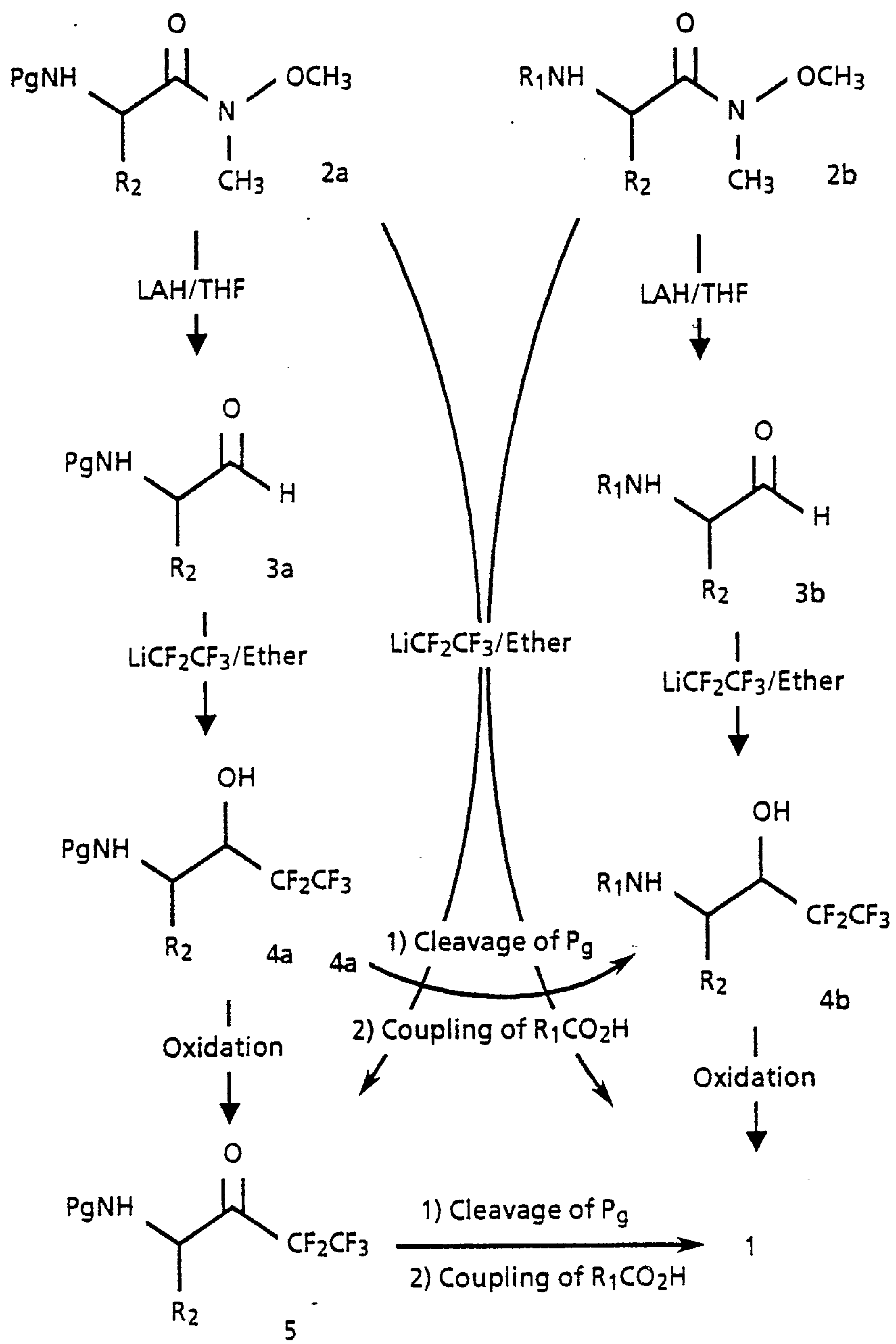

Specifically the compounds of this invention are prepared by reducing the N-methoxy-N-methyl amide of either formula 2a or 2b to produce the aldehydes of formulae 3a and 3b, respectively. Applicants prefer to use the compounds of formula 2a as the initial starting materials. The reduction can be performed in any way generally known and readily performed by those skilled in the art such as by use of lithium aluminum hydride (LAH). This reduction can be conveniently carried out by adding an excess of LAH to a cooled, typically about 0 ° C, solution of a formula 2a or 2b compound in a nonreactive solvent such as an ethereal solvent such as tetrahydrofuran (THF). After the reaction is substantially complete, typically after about 30 minutes, the reaction mixture is quenched by the addition of, for example, 10% potassium hydrogen sulfate and then water. The product can then be isolated by, for example, extraction of the aqueous mixture with a solvent such as ethyl acetate, drying and solvent removal. The crude product can be purified by, for example, column chromatography such as a silica gel column eluting with 55% ethyl acetate/hexane or recrystillazation.

The formulae 3a and 3b aldehydes are then reacted with the pentafluoroethyl anion, such as the lithium salt of the pentafluoroethyl anion to give the alcohols of formulae 4a or 4b, respectively. This condensation can be conveniently preformed by those skilled in the art by a modified procedure as described by P. G. Gassman and Neil J. O'Reilly, J. Org. Chem. 1987, 52, 2481-2490. In this procedure, the perfluoroethyl anion is generated *in situ* by addition of methyllithium/lithium bromide complex to a solution of the aldehyde and pentafluoroethyl iodide in a nonreactive solvent such as diethyl ether. The cooled (-78 ° - 0 ° C) reaction mixture is allowed to stir for about one-half to about 1 hour or until the reaction is substantially complete and then the mixture is quenched by pouring into an excess of dilute hydrochloric acid. The product is isolated by, for example, extraction with diethyl ether and subsequent solvent removal. The crude product is purified by, for example, chromatography on silica gel.

The alcohols of formulae 4a or 4b are then oxidized to give the amino-protected pentafluoroethyl ketone of formula 5 or the desired product of formula 1, respectively. The oxidation may be effected via the well-known Swern oxidation procedure, or with a modified Jones reaction using pyridinium dichromate, or a chromic anhydride-pyridinium complex, or with the Dess-Martin periodinane, 1,1,1-tris(acetyloxy)-1,1-dihydro-1,2-benzoiodoxol-3(1H)-one. Of course, if there are any protecting groups on the residues of the α-amino acid building blocks, such protecting groups may be removed after oxidation. The coupling procedures are effected according to standard procedures well known in the art.

In general the Swern oxidation is effected by reacting about 2 to 10 equivalents of dimethylsulfoxide (DMSO) with about 1 to 6 equivalents of trifluoroacetic anhydride [$(CF_3CO)_2O$] or oxalyl chloride [$(COCl)_2$], said reactants being dissolved in an inert solvent, e.g., methylene chloride ($CH_2Cl_2$], said reactor being under an inert atmosphere (e.g., nitrogen or equivalently functioning gas) under anhydrous conditions at temperatures of about -80 ° C to -50 ° C to form an *in situ* sulfonium adduct to which is added about 1 equivalent of an appropriate alcohol of formula 4a or 4b.

Preferably, the alcohols are dissolved in an inert solvent, e.g., $CH_2Cl_2$ or minimum amounts of DMSO, and the reaction mixture is allowed to warm to about -50 ° C (for about 10-20 minutes) and then the reaction is completed by adding about 3 to 10 equivalents of a tertiary amine, e.g., triethylamine, N-methylmorpholine, etc.

In general, the modified Jones oxidation procedure may conveniently be effected by reacting an alcohol of formula 4a or 4b with pyridinium dichromate by contacting the reactants together in a water-trapping molecular sieve powder, (e.g., a powdered 3 Angström molecular sieve), wherein said contact is in the presence of glacial acetic acid at about 0 ° C to 50 ° C, preferably at room temperature followed by isolation and then optionally removing amine protecting groups.

Alternatively, 1 to 5 equivalents of a chromic anhydride-pyridine complex (i.e., a Sarett reagent prepared *in situ* (see Fieser and Fieser "Reagents for Organic Synthesis" Vol. 1, pp. 145 and Sarett, et al., J.A.C.S. 25 , 422, (1953)) said complex being prepared *in situ* in an inert solvent (e.g., $CH_2Cl_2$) in an inert atmosphere under anhydrous conditions at 0 ° C to 50 ° C to which complex is added 1 equivalent of an alcohol of formula 4a or 4b allowing the reactants to interact for about 1 to 15 hours, followed by isolation and optionally removing amine protecting groups.

Another alternative process for converting an alcohol of formula 4a or 4b to the desired ketone of formula 1 or 5 is an oxidation reaction which employs Dess-Martin periodinane (see Dess Martin, J. Org. Chem. , 48 , 4155, (1983)). This oxidation is effected by contacting about 1 equivalent of the appropriate alcohol of formula 4a or 4b with 1 to 5 equivalents of periodinane (preferably 1.5 equivalents), said reagent being in suspension in an inert solvent (e.g., methylene chloride) under an inert atmosphere (preferably nitrogen) under anhydrous conditions at 0 ° C to 50 ° C (preferably room temperature) and allowing the reactants to interact for about 1 to 48 hours. Optional deprotection of the amine protecting groups may be effected as desired after the ketones have been isolated.

In the preferred mode of preparing the compounds of this invention the formula compounds are prepared by first converting the amino-protected, perfluoroethyl alcohol of formula 4a is converted to the corresponding compound of formula 4b, prior to final oxidation. The amino-protected, perfluoroethyl alcohol of formula 4a is first deprotected, if desired, and then any amino acids or peptide chain represented by $R_1$ can be added using standard α-amino acid or peptide coupling procedures. Where the $R_1$ group is made up of more than one amino acid, either the entire peptide chain can be added to the deprotected formual 4a compound or the amino acids can be coupled to the deprotected formula 4a compound sequentially. Alternatively, a combination of these two coupling methods can be used. In a like manner, the compounds of formula 5 can be converted to the desired formula 1 compounds.

In coupling individual amino acids or peptides to the deprotected formula 4a or formula 5 compounds, appropriate side chain protecting groups are employed. The selection and use of an appropriate protecting group for these side chain functionalities is within the ability of those skilled in the art and will depend upon the amino acid to be protected and the presence of other protected amino acid residues in the peptide. The selection of such a side chain protecting group is critical in that it must not be removed during the deprotection and coupling steps of the synthesis. For example, when Boc is used as the α-amino protecting group, the following side chain protecting groups are suitable: p -toluenesulfonyl (tosyl) moieties can be used to protect the amino side chains of amino acids such as Lys and Arg; p -methylbenzyl, acetamidomethyl, benzyl (Bzl), or t -butylsulfonyl moieties can be used to protect the sulfide containing side chains of amino acids such as cysteine, homocysteine, penicillamine and the like or derivatives thereof; benzyl (Bzl) or cyclohexyl ester moieties can be used to protect carboxylic acid side chains of amino acids such as Asp, Glu; a benzyl (Bzl) ether can be used to protect the hydroxy containing side chains of amino acids such as Ser and Thr; and a 2-bromocarbobenzoxy (2Br-Z) moiety can be used to protect the hydroxy containing side chains of amino acids such as Tyr. These side chain protecting groups are added and removed according to standard practices and procedures well known in the art. It is preferred to deprotect these side chain protecting groups with a solution of anisole in anhydrous hydrogen fluoride (1:10). Typically, deprotection of side chain protecting groups is performed after the peptide chain synthesis is complete but these groups can alternatively be removed at any other appropriate time. It is preferred to deprotect these side chains at the same time as the peptide is cleaved from the resin when solid phase synthetic methods are employed.

Alternatively, the compounds of formulae 2a and 2b can be converted directly to the compounds of formulae 5 or 1, respectively, by condensation of the N-methoxy-N-methyl amide with the lithium salt of the perfluoroethyl anion in the same manner in which the compounds of formulae 2a and 2b are converted to the compounds of formulae 3a and 3b, respectively.

The compounds are then isolated and purified by standard techniques. The desired amino acids, derivatives and isomers thereof can be obtained commercially or can be synthesized according to standard practices and procedures well known in the art.

The N-methoxy-N-methyl amides of formulae 2a and 2b are prepared from the corresponding α-amino acids of formulae 6a and 6b, respectively, in the usual manner. (See, for example, J.A. Fehrentz and B. Costra, Synthesis , 676-78 (1983).

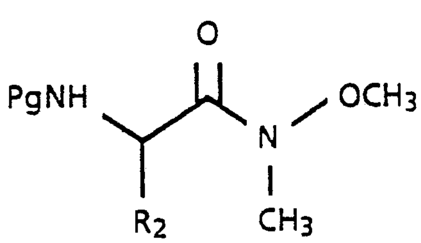

6a

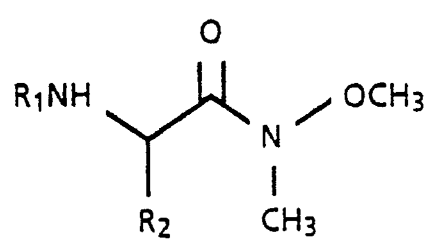

6b

Isobutylchloroformate is added to a cooled (i.e. -60 °C to about 0 °C) mixture of N-methylmorpholine or another sterically hindered, non-nucleophilic tertiary amine and a formulae 6a or 6b compound in a nonrective solvent such a methylene chloride. After about 5 minutes to about 1 hour, typically about 15 - 20 minutes, N,O-dimethylhydroxylamine HCl is added and the mixture allowed to stir for from about 30 minutes up to about 6 hours and then the reaction mixture is allowed to warm to room temperature. When the

reaction is substantially complete, typically after about 1 to about 10 hours, the mixture is poured into water and the aqueous phase is extracted with, for example, ethyl acetate. The desired compound is then isolated by solvent evaporation and crude purification can be accomplished by, for example, flash chromatography on silica gel eluting with ethyl acetate/hexane. Purification can be acomplished by, for example, flash chromatography on silica gel eluting with methylene chloride.

The following specific examples are given to illustrate the preparation of this invention although the scope of compounds is not meant to be limiting to the scope of compounds embraced by formula I.

## EXAMPLE 1

### PREPARATION OF CBZ-VaL-$C_2F_5$

A. Preparation of CBZ-Val[OH]-$CF_2CF_3$

A solution of Cbz-Val-H (0.55 g) in diethylether (8 ml) was cooled to -78° C and pentafluoroethyl iodide (0.5 ml) was added. To the mixture methyllithium-lithium bromide complex (2.8 ml of 1.5 M methyllithium-LiBr complex in diethyl ether) was added. The mixture was stirred at -78° C for 0.5 h, poured into dilute HCl and extracted with diethyl ether (2 x 100 ml). The combined extracts were dried over $Na_2SO_4$ followed by removal of solvent *in vacuo* to give crude product which was purified by flash chromatography to yield 97 mg of the expected product.
M.S. 356 for $M^+H$.

B. Preparation of CBZ-Val-$CF_2CF_3$

A solution of oxalyl chloride (0.03 ml) in methylene chloride (2 ml) was cooled to -55° C and dimethyl sulfoxide (0.08 ml) was added. The mixture was stirred for 5 min. and a solution of CBZVal[OH]-$CF_2CF_3$ (82 mg) dissolved in methylene chloride (1 ml) was added. The reaction mixture was stirred for 20 min. at -55° C, followed by the addition of triethylamine (0.5 ml) and warming to room temperature. The mixture was poured into $H_2O$ (100 ml) and extracted with ethyl acetate. The combined extracts were dried over $Na_2SO_4$ and the solvent was removed *in vacuo* to give the crude product which was purified by chromatography to yield 23 mg of the expected product.

## EXAMPLE 2

L-Phenylalaninamide, N-[(phenylmethoxy)carbonyl]-L-valyl-N-methoxy-N-methyl

To a suspension of L-phenylalanine, N-[N-[(phenylmethoxy)carbonyl]-L-valyl] (2.5 g, 6.25 mmol) in methylene chloride (25 ml), N-methylmorpholine (1.5 ml) was added. The solution was cooled to -15° C, followed by the addition of isobutyl chloroformate (0.8 ml]. The solution was stirred for 20min and N,O-dimethylhydroxylamine HCl (1.0 g) was added. The solution was stirred at -15° C for 1 h, allowed to warm to room temperature and stirred for an additional 3 h. The reaction mixture was poured into dil. $NaHCO_3$ and extracted with ethyl acetate (3 x 75 ml). The combined extracts were dried over $Na_2SO_4$, the solvent was removed *in vacuo* and the crude product was loaded onto a silica gel column for purification. The expected product was eluted with 75% EtOAc/hexane to yield 1.8 g.

## EXAMPLE 3

L-N-(Phenylmethoxy)carbonylphenylalaninamide, N′-methoxy-N′-methyl

To a solution of L-N-(phenylmethoxy)carbonylphenylalanine (25 g, 0.084 mol) in methylene chloride (300 ml), N-methylmorpoholine (18.4 ml, 0.167 mol) was added. The mixture was cooled to -15 °C and isobutyl chloroformate (10.8 ml, 83.6 mmol) was added. The mixture was stirred at -15 °C for 15 min followed by the addition of N,O-dimethylhydroxylamine HCl (8.5 g). The mixture was stirred at -15 °C for 1 h, allowed to warm to room temperature and stirred for 3 h. The reaction mixture was poured into $H_2O$ (300 ml) and the aqueous phase was extracted with methylene chloride (2 x 150 ml). The combined organic extracts were dried over $Na_2SO_4$, the volume was reduced to 100 ml and filtered through silica gel (2 inch). The silica gel was washed with methylene chloride (200 ml) and the solvent was removed from the combined filtrates to yield 26.14 g of the expected product.

## EXAMPLE 4

Carbamic acid, [5-[[(1,1-dimethylethoxy)carbonyl]amino]-6-(methoxymethylamino)-6-oxohexyl]-, phenylmethyl ester

A solution of L-lysine, $N^2$-[(1,1-dimethylethoxy]carbonyl]-$N^6$-[(phenylmethoxy)carbonyl] (10 g, 26.3 mmol) in methylene chloride was cooled to 0 °C and diisopropylethylamine (9.15 ml) was added. To the mixture isobutyl chloroformate (3.4 ml, 26.3 mmol) was added, followed by cooling to -15 °C, stirring for 15 min, followed by the addition of N,O-dimethylhydroxylamine HCl (2.7 g). The mixture was stirred at -15 °C for 2 h, allowed to warm to room temperature and stirred for 18 h. The reaction mixture was poured into $H_2O$ (200 ml) and extracted with methylene chloride (2 x 150 ml). The combined extracts were dried over $MgSO_4$ and removal of solvent *in vacuo* yielded 13.5 g crude product. The crude product (3.0 g) was loaded onto silica gel for purification. Elution with 50% EtOAc/hexane afforded 2.01 g of the expected product.

## EXAMPLE 5

L-Phenylalaninal, N[(phenylmethoxy)carbonyl]-L-valyl

A solution of L-phenylalaninamide, N[(phenylmethoxy)-carbonyl]-L-valyl-N′-methoxy-N′-methyl (3 g, 6.8 mol) in tetrahydrofuran (50 ml) was cooled to 0 °C and $LiAlH_4$ (250 mg) was added. The mixture was stirred at 0 °C for 30 min and quenched by the addition of 10% potassium hydrogen sulfate. The mixture was poured into $H_2O$ (400 ml) and the aqueous phase was extracted with ethyl acetate (3 x 150 ml). The combined organic extracts were dried over $MgSO_4$ and the solvent was removed *in vacuo*. The crude product was loaded onto silica gel for purification and the product was eluted with 55% EtOAc/hexane to yield 1.6 g of the expected compound.

## EXAMPLE 6

Preparation of BoC-Val-$CF_2CF_3$

1.0 g (3.8 mmol) of tBoc-Val-$N(OCH_3)CH_3$ was dissolved in 50 ml of diethyl ether and cooled to -78 °C. To the mixture 1.5 ml (12.2 mmol) pentafluoroethyliodide was added. To the mixture 6.0 ml (9.0 mmol] of 1.5 M lithiumbromide•methyllithium complex ($CH_3LiLiBr$) in diethyl ether was added. The mixture was stirred for 5 min. and checked by TLC. The reaction was incomplete. To the mixture 0.75 ml of pentafluoroethyliodide was added an additional 3.0 ml of $CH_3Li$•LiBr. Again reaction was incomplete so an additional 0.75 ml of pentafluoroethyliodide was added and 3.0 ml of $CH_3Li$•LiBr. Again reaction was about

75% complete by TLC analysis so another slug 0.75 ml of pentafluoroethyliodide and 3.0 ml of $CH_3Li \cdot LiBr$ were added. The mixture was stirred for 10 min., quenched into 200 ml of H2O and extracted with 2 x 150 ml of diethyl ether. the combined organic extracts were dried over $Na_2SO_4$, the solvent was removed *in vacuo* and the crude product loaded onto a 3 cm x 24 cm silica gel column and the product was eluted with 10% EtOAc/hex to yield 900 mg of product.

## EXAMPLE 7

Preparation of Cbz-Phe-$C_2F_5$

Cbz-Phe-N($OCH_3$)$CH_3$ (0.57 g, 1.7 mmol) was dissolved in 25 ml of diethyl ether and 0.75 ml (6.1 mmol) pentafluoroethyliodide was condensed and added to the cold mixture (approx. -55° C). To the mixture, 3.0 ml (4.5 mmol) of methyllithium•lithium bromide complex was added. The mixture was stirred for 15 min., the cold bath removed, and the mixture was stirred for 20 min. while warming to room temperature. The reaction mixture was then poured into dilute HCl and extracted with diethyl ether (3 x 50 ml). The combined extracts were dried over $Na_2SO_4$, the solvent was removed *in vacuo* and the crude product was loaded onto a silica gel column for purification (column 3 cm x 20 cm) the product was eluted with 20% EtOAc/hexane. The crude product was dissolved in 20% EtOAc/hexane and rechromatographed on a 2 cm/22 cm silica gel column, approx. 20 ml fractions were collected after void volume was discarded. Yield 285 mg.

## EXAMPLE 8

Preparation of Boc-Val-Pro-Val-$CF_2CF_3$

Boc-Val-$CF_2CF_3$ was dissolved in 100 ml of ethylacetate and the mixture was cooled to 0° C. The mixture was treated with HCl gas for 5 min., allowed to stir at 0° C for 20 min., (TLC analysis showed disappearance of starting material) and then the solvent was removed by rotary evaporation. The crude HCl salt was used without purificiation.

In a separate flask 0.54 g (1.7 mmol) of Boc-Val-Pro-OH was dissolved in a mixture of methylenechloride (6 ml) and n-methylmorpholine (0.55 ml, 5.1 mmol) and the mixture was cooled to -22° C. To the mixture 0.22 ml (1.7 mmol) of isobutyl chloroformate was added, the mixture was stirred at -22° C for 25 min., and then added to the HCl salt of 001E-190 (prepared as described in the above paragraph) which was suspended in 10 ml of CH2Cl2. The mixture was stirred for 1.5 h. The reaction mixture was then poured into 100 ml of $H_2O$ and extracted with 2 x 100 ml of diethyl ether. The combined organic extracts were washed with diluted HCl, diluted $NaHCo_3$ and then dried over $Na_2SO_4$. Removal of solvent gave 660 mg of crude compound. The product was purified by flash chromatography on a 13 cm x 24 cm silica gel column, with the product being eluted with 20% EtOAc/hex.

The foregoing describes in detail the generic and specific aspects of the scope of the invention as well as the manner of making and using the invention. In addition thereto, although such procedures are known in the art, references setting forth state of the art procedures by which the compounds may be evaluated for their biochemical effects are also included herein.

For example, human elastase is assayed *in vitro* using chromophoric peptides, succinylalanylalanylalanyl-p-nitroanilide (A1), methoxysuccinylalanylalanylprolylvalyl-p-nitroanilide (A2), and others, all of which are available commercially. The assay buffer, pH 8.0, and assay techniques are similar to those described by Lottenberg, et al. (A3, A4). Enzyme is purified from human sputum (A5), although recently it has become commercially available. Kinetic characterization of immediate inhibitors is by means of the Dixon plot (A6), whereas the characterization of slow- and/or tight-binding inhibitors used data analysis techniques reviewed by Williams and Morrison (A7).

Similarly, the other proteases are assayed and effects of inhibitors are assessed *in vitro* by similar spectroscopic techniques: cathepsin G (A2); thrombin (A3); chymotrypsin (A8); trypsin (A9); plasmin (A3); Cl esterase (A10); urokinase (A3); plasminogen activator (A11); acrosin (A12); beta-lactamase (A13); cathepsin

B (A14); pepsin (A15); cathepsin D (A16) and leucine aminopeptidase (A17). Pseudomonas elastase was measured in a coupled assay procedure using a human elastase substrate and microsomal aminopeptidase.

Radiometric assays of angiotensin I-converting enzyme and enkephalinase and their inhibitors were based on the procedure of Ryan (A18) and used tritiated substrates purchased from Ventrex Laboratories, Inc. Radioimmunoassay was used for studies with renin (A19). C3-convertase was measured as described by Tack, et al. (A20).

The individual assay references are elaborated upon by the following:

A1. The synthesis and analytical use of a highly sensitive and convenient substrate of elastase. J. Bieth, B. Spiess and C.G. Wermuth, Biochemical Medicine, 11 (1974) 350-375.

A2. Mapping the extended substrate binding site of cathepsin G and human leukocyte elastase. Studies with peptide substrates related to the alpha 1-protease inhibitor reactive site. K. Nakajima, J.C. Powers, B.M. Ashe and M. Zimmerman, The Journal of Biological Chemistry, 254 (1979) 4027-4032.

A3. Assay of coagulation proteases using peptide chromogenic and fluorogenic substrates. R. Lottenberg, U. Christensen, G.M. Jackson and P.L. Coleman, in Methods in Enzymology (L.Lorand, ed), Academic Press, New York, 1979, vol. 80, pp. 341-361.

A4. Solution composition dependent variation in extinction coefficients for p-nitroaniline. R. Lottenberg and C.M. Jackson, Biochimica et Biophysica Acta, 742 (1983) 558-564.

A5. A rapid procedure for the large scale purification of elastase and cathepsin G from human sputum. R.R. Martodam, R.J. Baugh, D.Y. Twumasi and I.E. Liener, Preparative Biochemistry, 9 (1979) 15-31.

A6. The determination of enzyme inhibitor constants. M. Dixon, The Biochemical Journal, 55 (1953) 170-171.

A7. The kinetics of reversible tight-binding inhibition. J.W.Williams and J.F. Morrison, in Methods in Enzymology (D.L. Purich, ed), Academic Press, New York, 1979, vol. 63, pp. 437-467.

A8. Two convenient spectrophotometric enzyme assays. A biochemistry experiment in kinetics. J.A. Hurlbut, T.N. Ball, H.C.Pound and J.L. Graves, Journal of Chemical Education, 50 (1973) 149-151.

A9. The preparation and properties of two new chromogenic substrates of trypsin. B.F. Erlanger, N. Kokowsky and W. Cohen, Archives of Biochemistry and Biophysics, 95 (1961) 271-278.

A10. The human complement system serine proteases Clr and Cls and their proenzymes. R.B. Sim, in Methods in Enzymology (L. Lorand, ed), Academic Press, New York, 1979, vol. 80, pp. 26-42.

A11. Extrinsic plasminogen activator and urokinase. J.H. Verheijen, C. Kluft, G.T.G. Chang and E. Mullaart, in Methods of Enzymatic Analysis (H.U. Bergmeyer, J. Bergmeyer and M. Grassl, eds), Verlag Chemie, Weinheim, 1984, third edition, vol. 5, pp. 425-433.

A12. Sperm acrosin. W. Mueller-Esterl and H. Fritz, in Methods in Enzymology (L. Lorand, ed), Academic Press, New York, 1979, vol. 80, pp. 621-632.

A13. Novel method for detection of beta-lactamases by using a chromogenic cephalosporin substrate. C.H. O'Callaghan, A. Morris, S.M. Kirby and A.K. Shingler, Antimicrobial Agents and Chemotherapy, 1 (1972) 283-288.

A14. Cathepsin B, cathepsin H, and cathepsin L. A.J. Barrett and H. Kirschke, in Methods in Enzymology (L. Lorand, ed), Academic Press, New York, 1979, vol. 80, pp. 535-561.

A15. Pepsins, gastricsins and their zymogens. A.P. Ryle, in Method of Enzymatic Analysis (H.U. Bergmeyer, J. Bergmeyer and M. Grassl, eds), Verlag Chemie, Weinheim, 1984, third edition, vol. 5, pp. 223-238.

A16. Cathepsin D, cathepsin E. V. Turk, T. Lah and I. Kregar, in Methods of Enzymatic Analysis (H.U. Bergmeyer, J. Bergmeyer and M. Grassl, eds), Verlag Chemie, Weinheim, 1984, third edition, vol 5, pp. 211-222.

A17. Amino acid arylamidase. J.C.M. Hafkenscheid, in Methods of Enzymatic Analysis (H.U. Bergmeyer, J. Bergmeyer and M. Grassl, eds), Verlag Chemie, Weinheim, 1984, third edition, vol. 5, pp. 11-15.

A18. Angiotensin I converting enzyme (kininase II). J.W. Ryan, in Methods of Enzymatic Analysis (H.U. Bergmeyer, J. Bergmeyer and M. Grassl, eds), Verlag Chemie, Weinheim, 1984, third edition, vol. 5, pp. 20-34.

A19. Renin. T. Inagami and M. Naruse, in Methods of Enzymatic Analysis (K.U. Bergmeyer, J. Bergmeyer and M. Grassl, eds), Verlag Chemie, Weinheim, 1984, third edition, vol. 5, pp. 249-258.

A20. The third, fourth, and fifth components of human complement: isolation and biochemical properties. B.F. Tack, J. Janatova, M.L. Thomas, R.A. Karrison and C.H. Hammer, in Methods in Enzymology (L. Lorand, ed), Academic Press, new York, 1979, vol. 870, pp. 64-101.

By following the techniques referenced above, as well as by utilization of other known techniques, as well as by comparison with compounds known to be useful for treatment of the above-mentioned disease states, it is believed that adequate material is available to enable one of ordinary skill in the art to practice

the invention. Of course, in the end-use application of the compounds of this invention, the compounds are preferably formulated into suitable pharmaceutical preparations such as tablets, capsules or elixers, for oral administration or in sterile solutions or suspensions for parenteral administration. The compounds of this invention can be administered to patients (animals and human) in need of such treatment in a dosage range of 5 to 500 mg per patient generally given several times, thus giving a total daily dose of from 5 to 2000 mg per day. As stated above, the dose will vary depending on severity of disease, weight of patient and other factors which a person skilled in the art will recognize.

Typically the compounds described above are formulated into pharmaceutical compositions as discussed below.

About 10 to 500 mg of a compound or mixture of compounds of formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may he present as coatings or to otherwise modify the physical form of the dosage unit. For instance, tablets may be coated with shellac, sugar or both. A syrup or elixer may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A compound of the formula (I)

**$R_1NHCH(R_2)COCF_2CF_3$** (I)

the hydrates, isosteres or the pharmaceutically acceptable salts thereof wherein

$R_1$ is hydrogen, an amino protecting group selected from Group K, an α-amino acid or a peptide comprised of a number of α-amino acids, each of said α-amino acid or peptide optionally bearing an amino protecting group preferably selected from Group K,

$R_2$ is the side chain of the α-amino acid responsible for directing the inhibitor to the active site of the enzyme wherein the said α-amino acids and peptide moieties are selected from Groups A, B, C, D, E, F, G and J, and K is a terminal amino protecting group, members of these groups being

Group A: Lys and Arg

B: Ser, Thr, Gln, Asn, Cys, His, and N-methyl derivatives

D: Pro, Ind

E: Ala, Leu, Ile, Val, Nva, Met, bVal, bAla, Nle and N-methyl derivatives

F: Phe, Tyr, Tyr(Me), Trp, Nal(1), and N-methyl derivatives

G: Gly, Sar J:

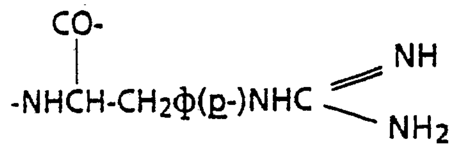 (J-1), 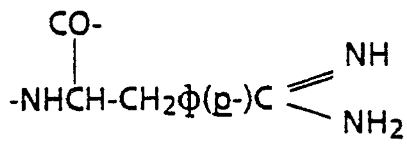 (J-2),

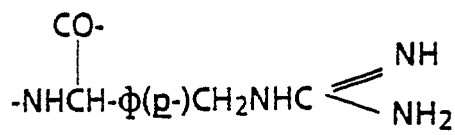 (J-3), and 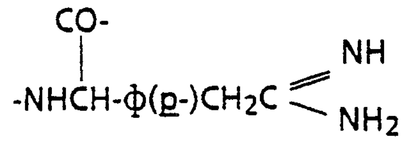 (J-4)

with Φ representing phenyl

K. Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulphonyl ($AdSO_2$), 1-Adamantaneacetyl (AdAc), 2-Carboxybenzoyl (2-Cbz), Phenylacetyl, t-Butylacetyl (Tba), bis [(1-naphthyl)methyl]-acetyl (BNMA),

or -A-$R_z$ wherein

A is 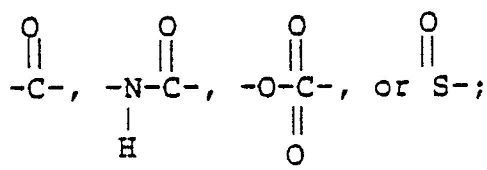 and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl) containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

2. A compound of claim 1 wherein
$R_1$ is -$P_2$-$P_3$-$P_4$-$P_5$ with
$P_2$ being an α-amino acid selected from Groups D, E and F,
$P_3$ is an α-amino acid of Group D, E, or lysine,
$P_4$ is an α-amino acid of Groups E or zero,
$P_5$ is a member of Group K,
$R_2$ is a side chain of an amino acid of Groups E and G.

3. A compound of claim 2 selected from the group consisting of
MeOSuc-Ala-Ala-Pro-Val-$C_2F_5$,
$AdSO_2$Lys(2Cbz)-Pro-Val-$C_2F_5$,
Cbz-Val-Pro-Val-$C_2F_5$,
ClΦSacBz-Val-Pro-Val-$C_2F_5$,
BrΦSacBz-Val-Pro-Val-$C_2F_5$,
Φ-SacBz-Val-Pro-Val-$C_2F_5$,
tPht-Val-Pro-Val-$C_2F_5$, and
Boc-Val-Pro-Val-$C_2F_5$.

4. A compound of claim 1 wherein
$R_1$ is -$P_2$-$P_3$-$P_4$-$P_5$ with
$P_2$ being selected from Groups D, E, or G,
$P_3$ is selected from Groups E or G,
$P_4$ is selected from Groups E, G or is deleted,
$P_5$ is a member of Group K,
$R_2$ is selected from a side chain of an amino acid of Groups E and F.

5. A compound of claim 4 selected from the group consisting of

MeOSuc-Ala-Ala-Pro-Phe-$C_2F_5$,
Suc-Ala-Ala-Pro-Phe-$C_2F_5$, and
ClΦSacBz-Val-Pro-Phe-$C_2F_5$.

6. A compound of claim 1 wherein
$R_1$ is (a)-$P_2$-$P_3$, (b) -$P_2$ or (c) -$P_2$-$P_3$-$P_4$ wherein
(a) $P_2$ is selected from Groups D, E or F,
$P_3$ is selected from Group F, each $P_3$ being in the D-configuration,
(b) $P_2$ is selected from Group K,
(c) $P_2$ is selected from Group E,
$P_3$ is selected from Groups C, G and E,
$P_4$ is selected from Groups F, G and E or is zero,
$R_2$ is the arginine side chain, or is selected from a side chain of an amino acid of Groups A and J.

7. A compound of claim 6 selected from the group consisting of
phe-Pro-NHCH(J-1)-$C_2F_5$,
phe-Pro-Arg-$C_2F_5$,
Dns-Arg-$C_2F_5$,
H-Phe-Ser-Ala-$C_2F_5$,
H-(D)-Phe-Pro-Lys-$C_2F_5$, and
Bz-NHCH(J-1)-$C_2F_5$.

8. A compound of claim 1 wherein
$R_1$ is -$P_2$-$P_3$-$P_4$-$P_5$ with
$P_2$ being selected from Groups D, E, G or K,
$P_3$ is selected from Groups E or G or K or is deleted,
$P_4$ is selected from Groups E or G or K or is deleted,
$P_5$ is selected from Group K or is deleted, and
$R_2$ is selected from a side chain of an amino acid of Groups E and F.

9. A compound of claim 8 selected from the group consisting of
Bz-Phe-$C_2F_5$,
Bz-Tyr-$C_2F_5$,
Ac-Leu-Phe-$C_2F_5$.

10. A compound of claim 1 wherein
$R_2$ is the arginine side chain, or is selected from a side chain of an amino acid of Groups A and J,
$R_1$ is selected from (a)-$P_2$-$P_3$, (b)-$P_2$ or (c)-$P_2$-$P_3$-$P_4$ with
(a) $P_2$ is selected from Groups E or F, $P_3$ is selected from from Group F, (each being in the D-configuration),
(b) $P_2$ is selected from Group K,
(c) $P_2$ is selected from Group D or E, $P_3$ is selected from Groups G and E, $P_4$ is selected from Groups G and E or is deleted.

11. A compound of claim 1 wherein
$R_1$ is -$P_2$-$P_3$-$P_4$ with
$P_2$ being selected from Group E or F,
$P_3$ is selected from Groups B, F or K, and
$P_4$ is selected from Group K,
$R_2$ is selected from a side chain of an amino acid of Groups A and J.

12. A compound of claim 11 selected from the group consisting of
Dns-Glu-Phe-Lys-$C_2F_5$,
Ac-Ala-NHCH(J-1)-$C_2F_5$, and
Ac-Ala-Lys-$C_2F_5$.

13. A compound of claim 1 wherein
$R_1$ generically is -$P_2$-$P_3$ with
$P_2$ being selected from Groups E, G, D, C, F, A or B,
$P_3$ is selected from Group K,
$R_2$ is selected from a side chain of an amino acid of Groups A and J.

14. A compound of claim 13 selected from the group consisting of
Cbz-Ala-Arg-$C_2F_5$ and
Ac-Ala-NHCH(J-1)CO-$C_2F_5$.

15. A compound of claim 1 wherein
$R_1$ is -$P_2$-$P_3$-$P_4$ with

$P_2$ being selected from Groups E or F,
$P_3$ is selected from Groups E or F, and
$P_4$ is selected from Group K,
$R_2$ is selected from a side chain of an amino acid of Groups A or J.

16. A compound of claim 1 which is
Bz-Leu-Ala-Arg-$C_2F_5$.

17. A compound of claim 1 wherein
$R_1$ is -$P_2$-$P_3$ with
$P_2$ being selected from Groups E and G, and
$P_3$ is selected from Group B,
$R_2$ is selected from a side chain of an amino acid of Groups A and J.

18. A compound of claim 17 selected from the group consisting of
K-Glu-Gly-Arg-$C_2F_5$ and
K-Glu-Gly-Phe(Gua)-$C_2F_5$,
wherein K is a protecting group selected from Group K.

19. A compound of claim 1 wherein
$R_1$ is -$P_2$-$P_3$-$P_4$ with
$P_2$ being selected from Group E or K,
$P_3$ is selected from Group E or is deleted,
$P_4$ is selected from Group K or is deleted,
$R_2$ is selected from a side chain of an amino acid of Groups A and J.

20. A compound of claim 19 selected from the group consisting of
Boc-Leu-Leu-Arg-$C_2F_5$,
Boc-Leu-Leu-Phe(Gua)-$C_2F_5$, and
Bz-NHCH(J-1)-$C_2F_5$.

21. A compound of claim 1 wherein
$R_1$ is $P_2$,
$P_2$ being selected from Group K,
$R_2$ is selected from a side chain of an amino acid of Groups G and C.

22. A compound of claim 21 selected from the group consisting of
$\Phi CH_2CONHCH_2CO$-$C_2F_5$, and
$\Phi CH_2CONHCH_2CHOH$-$C_2F_5$.

23. A compound of claim 1 wherein
$R_1$ is $P_2$-$P_3$ with
$P_2$ being Lys(Ac) or is selected from Groups E and C,
$P_3$ is selected from Group K,
$R_2$ is a methyl group.

24. A compound of claim 23 which is
Ac-Lys(Ac)-ala-$C_2F_5$.

25. A compound of claim 1 wherein
$R_1$ is -$P_2$-$P_3$-$P_4$-$P_5$-$P_6$ wherein
$P_2$ is selected from Groups E, C, or F,
$P_3$ is selected from Groups E or F or is deleted,
$P_4$ is selected from Groups E, D, F or is deleted,
$P_5$ is selected from Groups E, C, F or is deleted,
$P_6$ is selected from Group K or when $P_4$ is $\beta$-valine or $\beta$-alanine, $P_5$ and $P_6$ are deleted,
$R_2$ is selected from a side chain of an amino acid of Groups E or F or is cyclohexylmethylene.

26. A compound of claim 25 selected from the group consisting of
Cbz-Nal(1)-His-Leu-$C_2F_5$,
Cbz-Phe-His-Leu-$C_2F_5$,
Boc-Phe-Nva-Leu-$C_2F_5$,
Cbz-Phe-Nva-Leu-$C_2F_5$,
Boc-His-Pro-Phe-His-Leu-$C_2F_5$,
Cbz-Phe-His-Cha-$C_2F_5$,
Cbz-His-Leu-$C_2F_5$,
Boc-Phe-His-Leu-$C_2F_5$,
Boc-Phe-Nva-Cha-$C_2F_5$,
Boc-Tyr(Me)-Nva-Cha-$C_2F_5$,

Boc-Phe-Ala(3pyr)-Cha-$C_2F_5$,
Tba-Tyr(Me)-Nva-Cha-$C_2F_5$,
Tba-Tyr(Me)-Ala(4pyr)-Cha-$C_2F_5$,
bAla-Tyr(Me)-Nva-Cha-$C_2F_5$,
bVal-Tyr(Me)-Nva-Cha-$C_2F_5$,
bVal-Tyr(Me)-His-Cha-$C_2F_5$, and
bAla-Tyr(Me)-His-Cha-$C_2F_5$.

27. A compound of claim 1 wherein
$R_1$ is -$P_2$-$P_3$ wherein
$P_2$ is selected from group E,
$P_3$ is selected from Group K, and
$R_2$ is selected from a side chain of an amino acid of Group E.

28. A compound of claim 27 wherein
$R_1$ is MeOSuc-Ala- and
$R_2$ is a methyl group.

29. A compound of claim 1 wherein
$R_1$ is -$P_2$-$P_3$-$P_4$ with
$P_2$ being selected from Groups E or F,
$P_3$ is selected from Groups E or F,
$P_4$ is selected from Group K,
$R_2$ is selected from a side chain of an amino acid of Groups E and F.

30. A compound of claim 29 selected from the group consisting of
Iva-Val-Leu-$C_2F_5$ and
Iva-Val-Val-Leu-$C_2F_5$.

31. A compound of claim 1 wherein
$R_1$ is -$P_2$-$P_3$-$P_4$ with
$P_2$ being selected from Groups E and F,
$P_3$ is selected from Groups E and F or is deleted,
$P_4$ is selected from Group K,
$R_2$ is selected from a side chain of an amino acid of Groups E and F.

32. A compound of claim 31 selected from the group consisting of
Cbz-Val-Val-Phe-$C_2F_5$,
Iva-Val-Ala-Phe-$C_2F_5$, and
Iva-Val-Phe-$C_2F_5$.

33. A compound of claim 1 wherein
$R_1$ is selected from Group K, and
$R_2$ is selected from a side chain of an amino acid of Groups E, F and G.

34. A compound of claim 33 selected from the group consisting of
Bz-Phe-$C_2F_5$ and
Cbz-Phe-$C_2F_5$.

35. A compound of claim 1 wherein
$R_1$ is -$P_2$-$P_3$ with
$P_2$ being Gly and
$P_3$ being selected from Group F or is deleted, and
$R_2$ is H.

36. A compound of claim 1 which is
Tyr-Gly-Gly-$C_2F_5$.

37. A compound of claim 1 wherein
$R_1$ is -$P_2$-$P_3$ with
$P_2$ being selected from Group E,
$P_3$ is selected from Group K, and
$R_2$ is selected from a side chain of an amino acid of Groups E and G.

38. A compound of claim 37, said compound being MeOSuc-Ala-Ala-$C_2F_5$.

39. A compound of claim 1 wherein
$R_1$ is hydrogen, and
$R_2$ is selected from a side chain of an amino acid of Groups A, B, E, F and J.

40. A compound of claim 39 selected from the group consisting of
Leu-$C_2F_5$ and

Phe-$C_2F_5$.

41. A compound of claim 1 wherein
$R_1$ is $-P_2-P_3$ with
$P_2$ being selected from Groups E and F,
$P_3$ being selected from Groups C, E or F, the residues of which may be in either the D- or L-configuration, and
$R_2$ is selected from a side chain of an amino acid of Groups A or T.

42. A compound of claim 41 selected from the group consisting of
pro-Phe-Arg-$C_2F_5$, and
pro-Phe-NHCH(J-1)CO-$C_2F_5$.

43. A compound of claim 1 wherein
$R_1$ is $-P_2-P_3-P_4$ with
$P_2$ being selected from the Groups C, E, F and G,
$P_3$ being selected from the Groups C, E, F and G,
$P_4$ being selected from Group C, or being bAla or bVal, and optionally bearing an amino protecting group of Group K,
$R_2$ is a side chain of an amino acid of Groups F.

44. A compound of claim 43 selected from the group consisting of
Ser-Gln-Asn-Tyr-$C_2F_5$,
Ser-Gln-Asn-Phe-$C_2F_5$,
Ser-Leu-Asn-Tyr-$C_2F_5$,
Ser-Leu-Asn-Phe-$C_2F_5$,
Thr-Gln-Asn-Tyr-$C_2F_5$,
Thr-Gln-Asn-Phe-$C_2F_5$,
Thr-Leu-Asn-Tyr-$C_2F_5$,
Thr-Leu-Asn-Phe-$C_2F_5$,
Iva-Ser-Asn-Tyr-$C_2F_5$, and
Iva-Ser-Asn-Phe-$C_2F_5$.

45. A process for preparing a compound of the formula

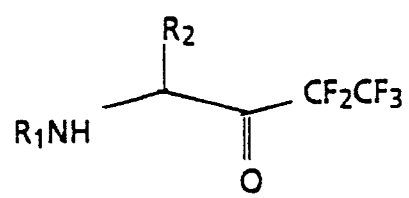

wherein $R_1$ and $R_2$ are as defined in Claim 1, which comprises oxidizing a compound of the Formula

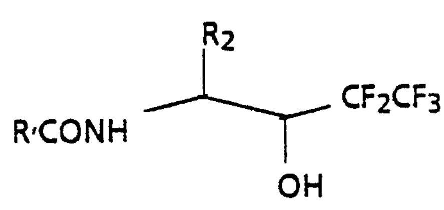

according to oxidation procedures (a), (b), (c) or (d) as follows:

a) preparing an *in situ* sulfonium adduct by reacting about 2 to 6 equivalents of dimethylsulfoxide with about 1 to 3 equivalents of $(CF_3CO)_2O$ or $(COCl)_2$; said reactants being dissolved in an inert solvent, under anhydrous conditions within the temperature range of about -80° C to -50° C, contacting said sulfonium adduct with about 1 equivalent of an alcohol of formula II, said alcohol being dissolved in an inert solvent or minimum amounts of dimethylsulfoxide, allowing the reactants to react at about -50° C for about 10 to 30 minutes, and completing the reaction by the addition of about 3 to 10 equivalents of a tertiary amine;
b) reacting an alcohol of formula II with pyridinium dichromate by contacting the reactants together in a powdered water-trapping molecular sieve in the presence of glacial acetic acid at about 0° C to 50° C;
c) reacting an alcohol of formula II with about 1 to 5 equivalents of a chromic anhydride-pyridine complex, said complex being formed *in situ* in an inert solvent under an inert atmosphere using anhydrous

conditions, said reaction of the alcohol being effected in the chromic anhydride-pyridine complex-reaction mixture for about 1 to 15 hours;
d) reacting an alcohol of formula II with 1,1,1-tris(acetyloxy)-1,1-dihydro-1,2-benziodoxol-3(1H)-one, said reaction being effected in an inert solvent under anhydrous conditions in an inert atmosphere for about 1 to 48 hours, reactions a, b, c or d being followed by an optional deprotection of any protected amine and optionally converting the obtained product to its pharmaceutically acceptable acid addition salt.
46. A pharmaceutical composition containing a compound of the formula (I) as defined in any of claims 1 to 44 or its hydrates, isosteres or pharmaceutically acceptable salts and optionally a pharmaceutically acceptable carrier, diluent and/or excipient.
47. The composition of claim 46 for inhibiting serine-, carboxylic acid- and metallo-proteinases.
48. Use of a compound of the formula (I) as defined in any of claims 1 to 44 or its hydrates, isosteres or pharmaceutically acceptable salts for the preparation of a pharmaceutical composition.
49. Use according to claim 48 wherein the composition is useful for inhibiting serine-, carboxylic acid- and metallo-proteinases.

Claims for the following Contracting State: ES

A process for preparing a compound of the formula (I)

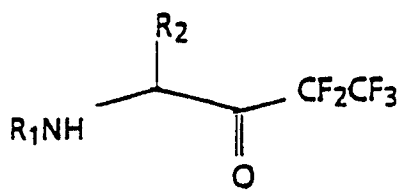

(I)

the hydrates, isosteres or the pharmaceutically acceptable salts thereof wherein
$R_1$ is hydrogen, an amino protecting group selected from Group K, an α-amino acid or a peptide comprised of a number of α-amino acids, each of said α-amino acid or peptide optionally bearing an amino protecting group preferably selected from Group K,
$R_2$ is the side chain of the α-amino acid responsible for directing the inhibitor to the active site of the enzyme wherein the said α-amino acids and peptide moieties are selected from Groups A, B, C, D, E, F, G and J, and K is a terminal amino protecting group, members of these groups being
Group A: Lys and Arg
B: Ser, Thr, Gln, Asn, Cys, His, and N-methyl derivatives
D: Pro, Ind
E: Ala, Leu, Ile, Val, Nva, Met, bVal, bAla, Nle and N-methyl derivatives
F: Phe, Tyr, Tyr(Me), Trp, Nal(1), and N-methyl derivatives
G: Gly, Sar
J:

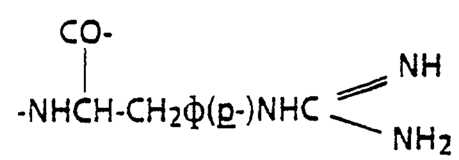 (J-1), 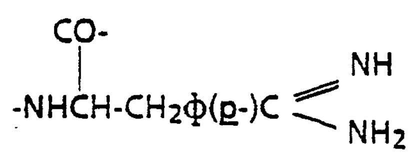 (J-2),

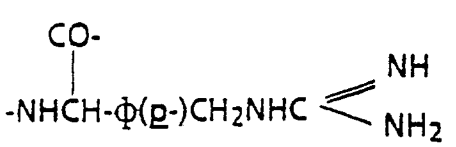 (J-3), and 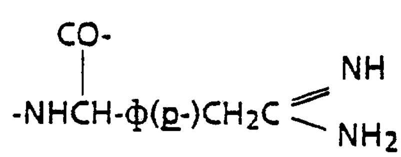 (J-4)

with Φ representing phenyl
K. Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulphonyl ($AdSO_2$), 1-Adaman-

taneacetyl (AdAc), 2-Carboxybenzoyl (2-Cbz), Phenylacetyl, t-Butylacetyl (Tba), bis [(1-naphthyl)methyl]-acetyl (BNMA),
or -A-$R_z$ wherein

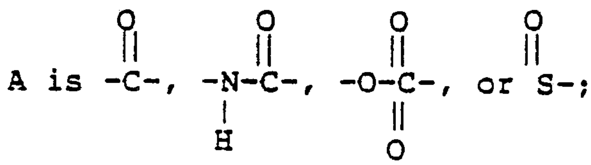

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl) containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto, which comprises oxidizing a compound of the Formula

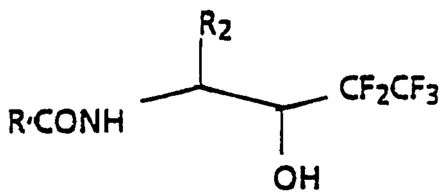

according to oxidation procedures (a), (b), (c) or (d) as follows:
a) preparing an *in situ* sulfonium adduct by reacting about 2 to 6 equivalents of dimethylsulfoxide with about 1 to 3 equivalents of $(CF_3CO)_2O$ or $(COCl)_2$; said reactants being dissolved in an inert solvent, under anhydrous conditions within the temperature range of about -80 °C to -50 °C, contacting said sulfonium adduct with about 1 equivalent of an alcohol of formula II, said alcohol being dissolved in an inert solvent or minimum amounts of dimethylsulfoxide, allowing the reactants to react at about -50 °C for about 10 to 30 minutes, and completing the reaction by the addition of about 3 to 10 equivalents of a tertiary amine;
b) reacting an alcohol of formula II with pyridinium dichromate by contacting the reactants together in a powdered water-trapping molecular sieve in the presence of glacial acetic acid at about 0 °C to 50 °C;
c) reacting an alcohol of formula II with about 1 to 5 equivalents of a chromic anhydride-pyridine complex, said complex being formed *in situ* in an inert solvent under an inert atmosphere using anhydrous conditions, said reaction of the alcohol being effected in the chromic anhydride-pyridine complex-reaction mixture for about 1 to 15 hours;
d) reacting an alcohol of formula II with 1,1,1-tris(acetyloxy)-1,1-dihydro-1,2-benziodoxol-3(1H)-one, said reaction being effected in an inert solvent under anhydrous conditions in an inert atmosphere for about 1 to 48 hours, reactions a, b, c or d being followed by an optional deprotection of any protected amine
and optionally converting the obtained product to its pharmaceutically acceptable acid addition salt.
2. The process according to claim 1 wherein compound (I) is $R_1$ is -$P_2$-$P_3$-$P_4$-$P_5$ with
$P_2$ being an $\alpha$-amino acid selected from Groups D, E and F,
$P_3$ is an $\alpha$-amino acid of Group D, E, or lysine,
$P_4$ is an $\alpha$-amino acid of Groups E or zero,
$P_5$ is a member of Group K,
$R_2$ is a side chain of an amino acid of Groups E and G.
3. The process according to claim 2 wherein compound (I) is selected from the group consisting of
MeOSuc-Ala-Ala-Pro-Val-$C_2F_5$,
$AdSO_2$-Lys(2Cbz)-Pro-Val-$C_2F_5$,
Cbz-Val-Pro-Val-$C_2F_5$,
ClΦSacBz-Val-Pro-Val-$C_2F_5$,
BrΦSacBz-Val-Pro-Val-$C_2F_5$,
ϕ-SacBz-Val-Pro-Val-$C_2F_5$,
tPht-Val-Pro-Val-$C_2F_5$, and

Boc-Val-Pro-Val-$C_2F_5$.

4. The process of claim 1 wherein
$R_1$ is $-P_2-P_3-P_4-P_5$ with
$P_2$ being selected from Groups D, E, or G,
$P_3$ is selected from Groups E or G,
$P_4$ is selected from Groups E, G or is deleted,
$P_5$ is a member of Group K,
$R_2$ is selected from a side chain of an amino acid of Groups E and F.

5. The process of claim 4 wherein compound (I) is selected from the group consisting of
MeOSuc-Ala-Ala-Pro-Phe-$C_2F_5$,
Suc-Ala-Ala-Pro-Phe-$C_2F_5$, and
ClΦSacBz-Val-Pro-Phe-$C_2F_5$.

6. The process of claim 1 wherein
$R_1$ is (a)-$P_2$-$P_3$, (b) -$P_2$ or (c) -$P_2$-$P_3$-$P_4$ wherein
(a) $P_2$ is selected from Groups D, E or F,
$P_3$ is selected from Group F, each $P_3$ being in the D-configuration,
(b) $P_2$ is selected from Group K,
(c) $P_2$ is selected from Group E,
$P_3$ is selected from Groups C, G and E,
$P_4$ is selected from Groups F, G and E or is zero,
$R_2$ is the arginine side chain, or is selected from a side chain of an amino acid of Groups A and J.

7. The process of claim 6 wherein compound (I) is selected from the group consisting of
phe-Pro-NHCH(J-1)-$C_2F_5$,
phe-Pro-Arg-$C_2F_5$,
Dns-Arg-$C_2F_5$,
H-Phe-Ser-Ala-$C_2F_5$,
H-(D)-Phe-Pro-Lys-$C_2F_5$, and
Bz-NHCH(J-1)-$C_2F_5$.

8. The process of claim 1 wherein
$R_1$ is $-P_2-P_3-P_4-P_5$ with
$P_2$ being selected from Groups D, E, G or K,
$P_3$ is selected from Groups E or G or K or is deleted,
$P_4$ is selected from Groups E or G or K or is deleted,
$P_5$ is selected from Group K or is deleted, and
$R_2$ is selected from a side chain of an amino acid of Groups E and F.

9. The process of claim 8 wherein compound (I) is selected from the group consisting of
Bz-Phe-$C_2F_5$,
Bz-Tyr-$C_2F_5$,
Ac-Leu-Phe-$C_2F_5$.

10. The process of claim 1 wherein
$R_2$ is the arginine side chain, or is selected from a side chain of an amino acid of Groups A and J,
$R_1$ is selected from (a)-$P_2$-$P_3$, (b)-$P_2$ or (c)-$P_2$-$P_3$-$P_4$ with
(a) $P_2$ is selected from Groups E or F, $P_3$ is selected from from Group F, (each being in the D-configuration),
(b) $P_2$ is selected from Group K,
(c) $P_2$ is selected from Group D or E, $P_3$ is selected from Groups G and E, $P_4$ is selected from Groups G and E or is deleted.

11. The process of claim 1 wherein
$R_1$ is $-P_2-P_3-P_4$ with
$P_2$ being selected from Group E or F,
$P_3$ is selected from Groups B, F or K, and
$P_4$ is selected from Group K,
$R_2$ is selected from a side chain of an amino acid of Groups A and J.

12. The process of claim 11 wherein compound (I) is selected from the group consisting of
Dns-Glu-Phe-Lys-$C_2F_5$,
Ac-Ala-NHCH(J-1)-$C_2F_5$, and
Ac-Ala-Lys-$C_2F_5$.

13. The process of claim 1 wherein

$R_1$ generically is $-P_2-P_3$ with
$P_2$ being selected from Groups E, G, D, C, F, A or B,
$P_3$ is selected from Group K,
$R_2$ is selected from a side chain of an amino acid of Groups A and J.
14. The process of claim 13 wherein compound (I) is selected from the group consisting of
Cbz-Ala-Arg-$C_2F_5$ and
Ac-Ala-NHCH(J-1)CO-$C_2F_5$.
15. The process of claim 1 wherein
$R_1$ is $-P_2-P_3-P_4$ with
$P_2$ being selected from Groups E or F,
$P_3$ is selected from Groups E or F, and
$P_4$ is selected from Group K,
$R_2$ is selected from a side chain of an amino acid of Groups A or J.
16. The process of claim 1 wherein compound (I) is Bz-Leu-Ala-Arg-$C_2F_5$.
17. The process of claim 1 wherein
$R_1$ is $-P_2-P_3$ with
$P_2$ being selected from Groups E and G, and
$P_3$ is selected from Group B,
$R_2$ is selected from a side chain of an amino acid of Groups A and J.
18. The process of claim 17 wherein compound (I) is selected from the group consisting of
K-Glu-Gly-Arg-$C_2F_5$ and
K-Glu-Gly-Phe(Gua)-$C_2F_5$,
wherein K is a protecting group selected from Group K.
19. The process of claim 1 wherein
$R_1$ is $-P_2-P_3-P_4$ with
$P_2$ being selected from Group E or K,
$P_3$ is selected from Group E or is deleted,
$P_4$ is selected from Group K or is deleted,
$R_2$ is selected from a side chain of an amino acid of Groups A and J.
20. The process of claim 19 wherein compound (I) is selected from the group consisting of
Boc-Leu-Leu-Arg-$C_2F_5$,
Boc-Leu-Leu-Phe(Gua)-$C_2F_5$, and
Bz-NHCH(J-1)-$C_2F_5$.
21. The process of claim 1 wherein
$R_1$ is $P_2$,
$P_2$ being selected from Group K,
$R_2$ is selected from a side chain of an amino acid of Groups E, G and C.
22. The process of claim 21 wherein compound (I) is selected from the group consisting of
$\Phi CH_2CONHCH_2CO-C_2F_5$, and
$\Phi CH_2CONHCH_2CHOH-C_2F_5$.
23. The process of claim 1 wherein
$R_1$ is $P_2-P_3$ with
$P_2$ being Lys(Ac) or is selected from Groups E and C,
$P_3$ is selected from Group K,
$R_2$ is a methyl group.
24. The process of claim 23 wherein compound (I) is
Ac-Lys(Ac)-ala-$C_2F_5$.
25. The process of claim 1 wherein
$R_1$ is $-P_2-P_3-P_4-P_5-P_6$ wherein
$P_2$ is selected from Groups E, C, or F,
$P_3$ is selected from Groups E or F or is deleted,
$P_4$ is selected from Groups E, D, F or is deleted,
$P_5$ is selected from Groups E, C, F or is deleted,
$P_6$ is selected from Group K or when $P_4$ is $\beta$-valine or $\beta$-alanine, $P_5$ and $P_6$ are deleted,
$R_2$ is selected from a side chain of an amino acid of Groups E or F or is cyclohexylmethylene.
26. The process of claim 25 wherein compound (I) is selected from the group consisting of
Cbz-Nal(1)-His-Leu-$C_2F_5$,
Cbz-Phe-His-Leu-$C_2F_5$,

Boc-Phe-Nva-Leu-$C_2F_5$,
Cbz-Phe-Nva-Leu-$C_2F_5$,
Boc-His-Pro-Phe-His-Leu-$C_2F_5$,
Cbz-Phe-His-Cha-$C_2F_5$,
Cbz-His-Leu-$C_2F_5$,
Boc-Phe-His-Leu-$C_2F_5$,
Boc-Phe-Nva-Cha-$C_2F_5$,
Boc-Tyr(Me)-Nva-Cha-$C_2F_5$,
Boc-Phe-Ala(3pyr)-Cha-$C_2F_5$,
Tba-Tyr(Me)-Nva-Cha-$C_2F_5$,
Tba-Tyr(Me)-Ala(4pyr)-Cha-$C_2F_5$,
bAla-Tyr(Me)-Nva-Cha-$C_2F_5$,
bVal-Tyr(Me)-Nva-Cha-$C_2F_5$,
bVal-Tyr(Me)-His-Cha-$C_2F_5$, and
bAla-Tyr(Me)-His-Cha-$C_2F_5$.

27. The process of claim 1 wherein
$R_1$ is -$P_2$-$P_3$ wherein
$P_2$ is selected from group E,
$P_3$ is selected from Group K, and
$R_2$ is selected from a side chain of an amino acid of Group E.

28. The process of claim 27 wherein
$R_1$ is MeOSuc-Ala- and
$R_2$ is a methyl group.

29. The process of claim 1 wherein
$R_1$ is -$P_2$-$P_3$-$P_4$ with
$P_2$ being selected from Groups E or F,
$P_3$ is selected from Groups E or F,
$P_4$ is selected from Group K,
$R_2$ is selected from a side chain of an amino acid of Groups E and F.

30. The process of claim 29 wherein compound (I) is selected from the group consisting of
Iva-Val-Leu-$C_2F_5$ and
Iva-Val-Val-Leu-$C_2F_5$.

31. The process of claim 1 wherein
$R_1$ is -$P_2$-$P_3$-$P_4$ with
$P_2$ being selected from Groups E and F,
$P_3$ is selected from Groups E and F or is deleted,
$P_4$ is selected from Group K,
$R_2$ is selected from a side chain of an amino acid of Groups E and F.

32. The process of claim 31 wherein compound (I) is selected from the group consisting of
Cbz-Val-Val-Phe-$C_2F_5$,
Iva-Val-Ala-Phe-$C_2F_5$, and
Iva-Val-Phe-$C_2F_5$.

33. The process of claim 1 wherein
$R_1$ is selected from Group K, and
$R_2$ is selected from a side chain of an amino acid of Groups E, F and G.

34. The process of claim 33 wherein compound (I) is selected from the group consisting of
Bz-Phe-$C_2F_5$ and
Cbz-Phe-$C_2F_5$.

35. The process of claim 1 wherein
$R_1$ is -$P_2$-$P_3$ with
$P_2$ being Gly and
$P_3$ being selected from Group F or is deleted, and $R_2$ is H.

36. The process of claim 1 wherein compound (I) is Tyr-Gly-Gly-$C_2F_5$.

37. The process of claim 1 wherein
$R_1$ is -$P_2$-$P_3$ with
$P_2$ being selected from Group E,
$P_3$ is selected from Group K, and
$R_2$ is selected from a side chain of an amino acid of Groups E and G.

38. The process of claim 37, wherein compound (I) is MeOSuc-Ala-Ala-$C_2F_5$.
39. The process of claim 1 wherein
$R_1$ is hydrogen, and
$R_2$ is selected from a side chain of an amino acid of Groups A, B, E, F and J.
40. The process of claim 39 wherein compound (I) is selected from the group consisting of
Leu-$C_2F_5$ and
Phe-$C_2F_5$.
41. The process of claim 1 wherein
$R_1$ is -$P_2$-$P_3$ with
$P_2$ being selected from Groups E and F,
$P_3$ being selected from Groups C, E or F, the residues of which may be in either the D- or L-configuration, and
$R_2$ is selected from a side chain of an amino acid of Groups A or T.
42. The process of claim 41 wherein compound (I) is selected from the group consisting of
pro-Phe-Arg-$C_2F_5$, and
pro-Phe-NHCH(J-1)CO-$C_2F_5$.
43. The process of claim 1 wherein
$R_1$ is -$P_2$-$P_3$-$P_4$ with
$P_2$ being selected from the Groups C, E, F and G,
$P_3$ being selected from the Groups C, E, F and G,
$P_4$ being selected from Group C, or being bAla or bVal, and optionally bearing an amino protecting group of Group K,
$R_2$ is a side chain of an amino acid of Groups F.
44. The process of claim 43 wherein compound (I) is selected from the group consisting of
Ser-Gln-Asn-Tyr-$C_2F_5$,
Ser-Gln-Asn-Phe-$C_2F_5$,
Ser-Leu-Asn-Tyr-$C_2F_5$,
Ser-Leu-Asn-Phe-$C_2F_5$,
Thr-Gln-Asn-Tyr-$C_2F_5$,
Thr-Gln-Asn-Phe-$C_2F_5$,
Thr-Leu-Asn-Tyr-$C_2F_5$,
Thr-Leu-Asn-Phe-$C_2F_5$,
Iva-Ser-Asn-Tyr-$C_2F_5$, and
Iva-Ser-Asn-Phe-$C_2F_5$.

Claims for the following Contracting State: GR

1. A compound of the formula (I)
**$R_1NHCH(R_2)COCF_2CF_3$** (I)
the hydrates, isosteres or the pharmaceutically acceptable salts thereof wherein
$R_1$ is hydrogen, an amino protecting group selected from Group K, an $\alpha$-amino acid or a peptide comprised of a number of $\alpha$-amino acids, each of said $\alpha$-amino acid or peptide optionally bearing an amino protecting group preferably selected from Group K,
$R_2$ is the side chain of the $\alpha$-amino acid responsible for directing the inhibitor to the active site of the enzyme wherein the said $\alpha$-amino acids and peptide moieties are selected from Groups A, B, C, D, E, F, G and J, and K is a terminal amino protecting group, members of these groups being
Group A: Lys and Arg
B: Ser, Thr, Gln, Asn, Cys, His, and N-methyl derivatives
D: Pro, Ind
E: Ala, Leu, Ile, Val, Nva, Met, bVal, bAla, Nle and N-methyl derivatives
F: Phe, Tyr, Tyr(Me), Trp, Nal(1), and N-methyl derivatives
G: Gly, Sar
J:

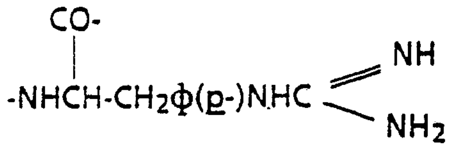 (J-1), 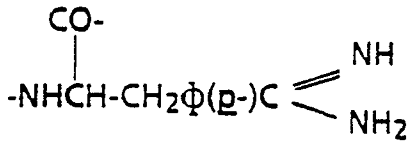 (J-2),

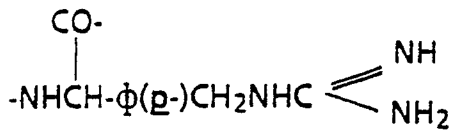 (J-3), and 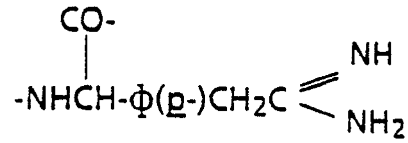 (J-4)

with Φ representing phenyl

K. Acetyl (Ac), Succinyl (Suc), Benzoyl (Bz), t-Butyloxycarbonyl (Boc), Carbobenzyloxy (Cbz), Tosyl (Ts), Dansyl (Dns), Isovaleryl (Iva), Methoxysuccinyl (MeOSuc), 1-Adamantanesulphonyl ($AdSO_2$), 1-Adamantaneacetyl (AdAc), 2-Carboxybenzoyl (2-Cbz), Phenylacetyl, t-Butylacetyl (Tba), bis [(1-naphthyl)methyl]-acetyl (BNMA),

or $-A-R_z$ wherein

A is 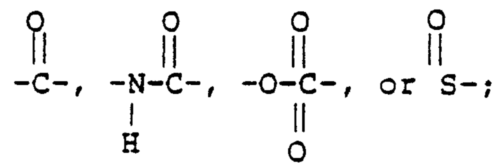

and

$R_z$ is an aryl group containing 6, 10 or 12 carbons suitably substituted by 1 to 3 members selected independently from the group consisting of fluoro, chloro, bromo, iodo, trifluoromethyl, hydroxy, alkyl containing from 1 to 6 carbons, alkoxy containing from 1 to 6 carbons, carboxy, alkylcarbonylamino wherein the alkyl group contains 1 to 6 carbons, 5-tetrazolyl, and acylsulfonamido (i.e., acylaminosulfonyl and sulfonylaminocarbonyl) containing from 1 to 15 carbons, provided that when the acylsulfonamido contains an aryl the aryl may be further substituted by a member selected from fluoro, chloro, bromo, iodo and nitro; and such other terminal amino protecting groups which are functionally equivalent thereto.

2. A compound of claim 1 wherein
$R_1$ is $-P_2-P_3-P_4-P_5$ with
$P_2$ being an α-amino acid selected from Groups D, E and F,
$P_3$ is an α-amino acid of Group D, E, or lysine,
$P_4$ is an α-amino acid of Groups E or zero,
$P_5$ is a member of Group K,
$R_2$ is a side chain of an amino acid of Groups E and G.

3. A compound of claim 2 selected from the group consisting of
MeOSuc-Ala-Ala-Pro-Val-$C_2F_5$,
$AdSO_2$-Lys(2Cbz)-Pro-Val-$C_2F_5$,
Cbz-Val-Pro-Val-$C_2F_5$,
ClφSacBz-Val-Pro-Val-$C_2F_5$,
BrΦSacBz-Val-Pro-Val-$C_2F_5$,
φ-SacBz-Val-Pro-Val-$C_2F_5$,
tPht-Val-Pro-Val-$C_2F_5$, and
Boc-Val-Pro-Val-$C_2F_5$.

4. A compound of claim 1 wherein
$R_1$ is $-P_2-P_3-P_4-P_5$ with
$P_2$ being selected from Groups D, E, or G,
$P_3$ is selected from Groups E or G,
$P_4$ is selected from Groups E, G or is deleted,
$P_5$ is a member of Group K,
$R_2$ is selected from a side chain of an amino acid of Groups E and F.

5. A compound of claim 4 selected from the group consisting of
MeOSuc-Ala-Ala-Pro-Phe-$C_2F_5$,
Suc-Ala-Ala-Pro-Phe-$C_2F_5$, and
ClΦSacBz-Val-Pro-Phe-$C_2F_5$.

6. A compound of claim 1 wherein
$R_1$ is (a)-$P_2$-$P_3$, (b) -$P_2$ or (c) -$P_2$-$P_3$-$P_4$ wherein
(a) $P_2$ is selected from Groups D, E or F,
$P_3$ is selected from Group F, each $P_3$ being in the D-configuration,
(b) $P_2$ is selected from Group K,
(c) $P_2$ is selected from Group E,
$P_3$ is selected from Groups C, G and E,
$P_4$ is selected from Groups F, G and E or is zero,
$R_2$ is the arginine side chain, or is selected from a side chain of an amino acid of Groups A and J.

7. A compound of claim 6 selected from the group consisting of
phe-Pro-NHCH(J-1)-$C_2F_5$,
phe-Pro-Arg-$C_2F_5$,
Dns-Arg-$C_2F_5$,
H-Phe-Ser-Ala-$C_2F_5$,
H-(D)-Phe-Pro-Lys-$C_2F_5$, and
Bz-NHCH(J-1)-$C_2F_5$.

8. A compound of claim 1 wherein
$R_1$ is -$P_2$-$P_3$-$P_4$-$P_5$ with
$P_2$ being selected from Groups D, E, G or K,
$P_3$ is selected from Groups E or G or K or is deleted,
$P_4$ is selected from Groups E or G or K or is deleted,
$P_5$ is selected from Group K or is deleted, and
$R_2$ is selected from a side chain of an amino acid of Groups E and F.

9. A compound of claim 8 selected from the group consisting of
Bz-Phe-$C_2F_5$,
Bz-Tyr-$C_2F_5$,
Ac-Leu-Phe-$C_2F_5$.

10. A compound of claim 1 wherein
$R_2$ is the arginine side chain, or is selected from a side chain of an amino acid of Groups A and J,
$R_1$ is selected from (a)-$P_2$-$P_3$, (b)-$P_2$ or (c)-$P_2$-$P_3$-$P_4$ with
(a) $P_2$ is selected from Groups E or F, $P_3$ is selected from from Group F, (each being in the D-configuration), (b) $P_2$ is selected from Group K,
(c) $P_2$ is selected from Group D or E, $P_3$ is selected from Groups G and E, $P_4$ is selected from Groups G and E or is deleted.

11. A compound of claim 1 wherein
$R_1$ is -$P_2$-$P_3$-$P_4$ with
$P_2$ being selected from Group E or F,
$P_3$ is selected from Groups B, F or K, and
$P_4$ is selected from Group K,
$R_2$ is selected from a side chain of an amino acid of Groups A and J.

12. A compound of claim 11 selected from the group consisting of
Dns-Glu-Phe-Lys-$C_2F_5$,
Ac-Ala-NHCH(J-1)-$C_2F_5$, and
Ac-Ala-Lys-$C_2F_5$.

13. A compound of claim 1 wherein
$R_1$ generically is -$P_2$-$P_3$ with
$P_2$ being selected from Groups E, G, D, C, F, A or B,
$P_3$ is selected from Group K,
$R_2$ is selected from a side chain of an amino acid of Groups A and J.

14. A compound of claim 13 selected from the group consisting of
Cbz-Ala-Arg-$C_2F_5$ and
Ac-Ala-NHCH(J-1)CO-$C_2F_5$.

15. A compound of claim 1 wherein
$R_1$ is -$P_2$-$P_3$-$P_4$ with

$P_2$ being selected from Groups E or F,
$P_3$ is selected from Groups E or F, and
$P_4$ is selected from Group K,
$R_2$ is selected from a side chain of an amino acid of Groups A or J.

16. A compound of claim 1 which is
Bz-Leu-Ala-Arg-$C_2F_5$.

17. A compound of claim 1 wherein
$R_1$ is -$P_2$-$P_3$ with
$P_2$ being selected from Groups E and G, and
$P_3$ is selected from Group B,
$R_2$ is selected from a side chain of an amino acid of Groups A and J.

18. A compound of claim 17 selected from the group consisting of
K-Glu-Gly-Arg-$C_2F_5$ and
K-Glu-Gly-Phe(Gua)-$C_2F_5$,
wherein K is a protecting group selected from Group K.

19. A compound of claim 1 wherein
$R_1$ is -$P_2$-$P_3$-$P_4$ with
$P_2$ being selected from Group E or K,
$P_3$ is selected from Group E or is deleted,
$P_4$ is selected from Group K or is deleted,
$R_2$ is selected from a side chain of an amino acid of Groups A and J.

20. A compound of claim 19 selected from the group consisting of
Boc-Leu-Leu-Arg-$C_2F_5$,
Boc-Leu-Leu-Phe(Gua)-$C_2F_5$, and
Bz-NHCH(J-1)-$C_2F_5$.

21. A compound of claim 1 wherein
$R_1$ is $P_2$,
$P_2$ being selected from Group K,
$R_2$ is selected from a side chain of an amino acid of Groups E, G and C.

22. A compound of claim 21 selected from the group consisting of
$\Phi CH_2CONHCH_2CO-C_2F_5$, and
$\Phi CH_2CONHCH_2CHOH-C_2F_5$.

23. A compound of claim 1 wherein
$R_1$ is $P_2$-$P_3$ with
$P_2$ being Lys(Ac) or is selected from Groups E and C,
$P_3$ is selected from Group K,
$R_2$ is a methyl group.

24. A compound of claim 23 which is
Ac-Lys(Ac)-ala-$C_2F_5$.

25. A compound of claim 1 wherein
$R_1$ is -$P_2$-$P_3$-$P_4$-$P_5$-$P_6$ wherein
$P_2$ is selected from Groups E, C, or F,
$P_3$ is selected from Groups E or F or is deleted,
$P_4$ is selected from Groups E, D, F or is deleted,
$P_5$ is selected from Groups E, C, F or is deleted,
$P_6$ is selected from Group K or when $P_4$ is $\beta$-valine or $\beta$-alanine, $P_5$ and $P_6$ are deleted,
$R_2$ is selected from a side chain of an amino acid of Groups E or F or is cyclohexylmethylene.

26. A compound of claim 25 selected from the group consisting of
Cbz-Nal(1)-His-Leu-$C_2F_5$,
Cbz-Phe-His-Leu-$C_2F_5$,
Boc-Phe-Nva-Leu-$C_2F_5$,
Cbz-Phe-Nva-Leu-$C_2F_5$,
Boc-His-Pro-Phe-His-Leu-$C_2F_5$,
Cbz-Phe-His-Cha-$C_2F_5$,
Cbz-His-Leu-$C_2F_5$,
Boc-Phe-His-Leu-$C_2F_5$,
Boc-Phe-Nva-Cha-$C_2F_5$,
Boc-Tyr(Me)-Nva-Cha-$C_2F_5$,

Boc-Phe-Ala(3pyr)-Cha-$C_2F_5$,
Tba-Tyr(Me)-Nva-Cha-$C_2F_5$,
Tba-Tyr(Me)-Ala(4pyr)-Cha-$C_2F_5$,
bAla-Tyr(Me)-Nva-Cha-$C_2F_5$,
bVal-Tyr(Me)-Nva-Cha-$C_2F_5$,
bVal-Tyr(Me)-His-Cha-$C_2F_5$, and
bAla-Tyr(Me)-His-Cha-$C_2F_5$.

27. A compound of claim 1 wherein
$R_1$ is $-P_2-P_3$ wherein
$P_2$ is selected from group E,
$P_3$ is selected from Group K, and
$R_2$ is selected from a side chain of an amino acid of Group E.

28. A compound of claim 27 wherein
$R_1$ is MeOSuc-Ala- and
$R_2$ is a methyl group.

29. A compound of claim 1 wherein
$R_1$ is $-P_2-P_3-P_4$ with
$P_2$ being selected from Groups E or F,
$P_3$ is selected from Groups E or F,
$P_4$ is selected from Group K,
$R_2$ is selected from a side chain of an amino acid of Groups E and F.

30. A compound of claim 29 selected from the group consisting of
Iva-Val-Leu-$C_2F_5$ and
Iva-Val-Val-Leu-$C_2F_5$.

31. A compound of claim 1 wherein
$R_1$ is $-P_2-P_3-P_4$ with
$P_2$ being selected from Groups E and F,
$P_3$ is selected from Groups E and F or is deleted,
$P_4$ is selected from Group K,
$R_2$ is selected from a side chain of an amino acid of Groups E and F.

32. A compound of claim 31 selected from the group consisting of
Cbz-Val-Val-Phe-$C_2F_5$,
Iva-Val-Ala-Phe-$C_2F_5$, and
Iva-Val-Phe-$C_2F_5$.

33. A compound of claim 1 wherein
$R_1$ is selected from Group K, and
$R_2$ is selected from a side chain of an amino acid of Groups E, F and G.

34. A compound of claim 33 selected from the group consisting of
Bz-Phe-$C_2F_5$ and
Cbz-Phe-$C_2F_5$.

35. A compound of claim 1 wherein
$R_1$ is $-P_2-P_3$ with
$P_2$ being Gly and
$P_3$ being selected from Group F or is deleted, and $R_2$ is H.

36. A compound of claim 1 which is
Tyr-Gly-Gly-$C_2F_5$.

37. A compound of claim 1 wherein
$R_1$ is $-P_2-P_3$ with
$P_2$ being selected from Group E,
$P_3$ is selected from Group K, and
$R_2$ is selected from a side chain of an amino acid of Groups E and G.

38. A compound of claim 37, said compound being MeOSuc-Ala-Ala-$C_2F_5$.

39. A compound of claim 1 wherein
$R_1$ is hydrogen, and
$R_2$ is selected from a side chain of an amino acid of Groups A, B, E, F and J.

40. A compound of claim 39 selected from the group consisting of
Leu-$C_2F_5$ and
Phe-$C_2F_5$.

41. A compound of claim 1 wherein
$R_1$ is $-P_2-P_3$ with
$P_2$ being selected from Groups E and F,
$P_3$ being selected from Groups C, E or F, the residues of which may be in either the D- or L-configuration, and
$R_2$ is selected from a side chain of an amino acid of Groups A or T.
42. A compound of claim 41 selected from the group consisting of
pro-Phe-Arg-$C_2F_5$, and
pro-Phe-NHCH(J-1)CO-$C_2F_5$.
43. A compound of claim 1 wherein
$R_1$ is $-P_2-P_3-P_4$ with
$P_2$ being selected from the Groups C, E, F and G,
$P_3$ being selected from the Groups C, E, F and G,
$P_4$ being selected from Group C, or being bAla or bVal, and optionally bearing an amino protecting group of Group K,
$R_2$ is a side chain of an amino acid of Groups F.
44. A compound of claim 43 selected from the group consisting of
Ser-Gln-Asn-Tyr-$C_2F_5$,
Ser-Gln-Asn-Phe-$C_2F_5$,
Ser-Leu-Asn-Tyr-$C_2F_5$,
Ser-Leu-Asn-Phe-$C_2F_5$,
Thr-Gln-Asn-Tyr-$C_2F_5$,
Thr-Gln-Asn-Phe-$C_2F_5$,
Thr-Leu-Asn-Tyr-$C_2F_5$,
Thr-Leu-Asn-Phe-$C_2F_5$,
Iva-Ser-Asn-Tyr-$C_2F_5$, and
Iva-Ser-Asn-Phe-$C_2F_5$.
45. A process for preparing a compound of the formula

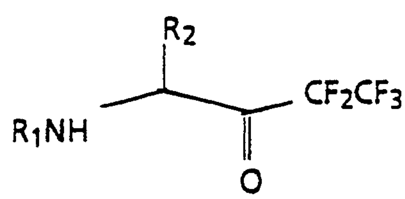

wherein $R_1$ and $R_2$ are as defined in Claim 1, which comprises oxidizing a compound of the Formula

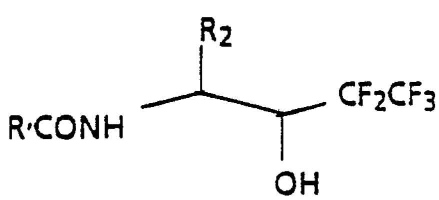

according to oxidation procedures (a), (b), (c) or (d) as follows:
a) preparing an *in situ* sulfonium adduct by reacting about 2 to 6 equivalents of dimethylsulfoxide with about 1 to 3 equivalents of $(CF_3CO)_2O$ or $(COCl)_2$; said reactants being dissolved in an inert solvent, under anhydrous conditions within the temperature range of about -80° C to -50° C, contacting said sulfonium adduct with about 1 equivalent of an alcohol of formula II, said alcohol being dissolved in an inert solvent or minimum amounts of dimethylsulfoxide, allowing the reactants to react at about -50° C for about 10 to 30 minutes, and completing the reaction by the addition of about 3 to 10 equivalents of a tertiary amine;
b) reacting an alcohol of formula II with pyridinium dichromate by contacting the reactants together in a powdered water-trapping molecular sieve in the presence of glacial acetic acid at about 0° C to 50° C;
c) reacting an alcohol of formula II with about 1 to 5 equivalents of a chromic anhydride-pyridine complex, said complex being formed *in situ* in an inert solvent under an inert atmosphere using anhydrous

conditions, said reaction of the alcohol being effected in the chromic anhydride-pyridine complex-reaction mixture for about 1 to 15 hours;

d) reacting an alcohol of formula II with 1,1,1-tris(acetyloxy)-1,1-dihydro-1,2-benzodoxol-3(1H)-one, said reaction being effected in an inert solvent under anhydrous conditions in an inert atmosphere for about 1 to 48 hours, reactions a, b, c or d being followed by an optional deprotection of any protected amine and optionally converting the obtained product to its pharmaceutically acceptable acid addition salt.

46. Use of a compound of the formula (I) as defined in any of claims 1 to 44 or its hydrates, isosteres or pharmaceutically acceptable salts for the preparation of a pharmaceutical composition.

47. Use according to claim 48 wherein the composition is useful for inhibiting serine-, carboxylic acid- and metallo-proteinases.